# EUROPEAN PATENT SPECIFICATION

(11) **EP 2 828 660 B1**
(45) Date of publication and mention of the grant of the patent: **06.12.2017**
(21) Application number: 13720082.0
(22) Date of filing: 20.03.2013
(51) Int. Cl.: G01N 33/543, G01N 33/68, G01N 33/564

(54) **A METHOD AND A KIT FOR THE DETECTION OF ANTI-BETA AMYLOID ANTIBODIES**
VERFAHREN UND TESTSATZ ZUM NACHWEIS VON ANTI-BETA-AMYLOID-ANTIKÖRPERN
PROCÉDÉ ET KIT POUR LA DÉTECTION D'ANTICORPS ANTI-BÊTA AMYLOÏDES

(30) Priority: 20.03.2012 IT RM20120103; 02.08.2012 IT RM20120383
(43) Date of publication of application: 28.01.2015
(73) Proprietor: Università degli Studi di Milano - Bicocca, 20126 Milano (IT)
(72) Inventor: PIAZZA, Fabrizio, I-20900 Monza MB (IT); FERRARESE, Carlo, I-20900 Monza MB (IT)
(74) Representative: Di Giovine, Paolo
(86) International application number: PCT/IB2013/052206
(87) International publication number: WO 2013/140349

(56) References cited:
- WO-A1-2010/128139
- WO-A1-2011/033046
- BRETTSCHNEIDER S ET AL: "Decreased serum amyloid beta1-42 autoantibody levels in Alzheimer's disease, determined by a newly developed immuno-precipitation assay with radiolabeled amyloid beta1-42 peptide", BIOLOGICAL PSYCHIATRY, ELSEVIER SCIENCE, NEW YORK, NY, US, vol. 57, no. 7, 1 April 2005 (2005-04-01), pages 813-816, XP027734981, ISSN: 0006-3223 [retrieved on 2005-04-01]
- RICHARD D. HOLLISTER ET AL: "Immunohistochemical localization of tissue factor pathway inhibitor-1 (TFPI-1), a Kunitz proteinase inhibitor, in Alzheimer's disease", BRAIN RESEARCH, vol. 728, no. 1, 1 July 1996 (1996-07-01), pages 13-19, XP055041732, ISSN: 0006-8993, DOI: 10.1016/0006-8993(96)00384-8
- FABRIZIO PIAZZA ET AL: "Increased tissue factor pathway inhibitor and homocysteine in Alzheimer's disease", NEUROBIOLOGY OF AGING, vol. 33, no. 2, 1 February 2012 (2012-02-01), pages 226-233, XP055041731, ISSN: 0197-4580, DOI: 10.1016/j.neurobiolaging.2010.02.016
- GRUDEN M A ET AL: "AUTOIMMUNE RESPONSES TO AMYLOID STRUCTURES OF ABETA(25-35) PEPTIDE AND HUMAN LYSOZYME IN THE SERUM OF PATIENTS WITH PROGRESSIVE ALZHEIMER'S DISEASE", DEMENTIA AND GERIATRIC COGNITIVE DISORDERS, KARGER, BASEL, CH, vol. 18, no. 2, 1 January 2004 (2004-01-01), pages 165-171, XP008056285, ISSN: 1420-8008, DOI: 10.1159/000079197
- IRINA PERDIVARA ET AL: "Glycosylation profiles of epitope-specific anti-beta-amyloid antibodies revealed by liquid chromatography-mass spectrometry", GLYCOBIOLOGY, OXFORD UNIVERSITY PRESS, US, vol. 19, no. 9, 1 September 2009 (2009-09-01), pages 958-970, XP002621663, ISSN: 0959-6658, DOI: 10.1093/GLYCOB/CWP038 [retrieved on 2009-03-24]

## Description

The present description relates to an in *vitro* method for the determination of antibodies anti-beta amyloid protein and of endothelial damage (by dosage of biomarker Tissue Factor Pathway Inhibitor, TFPI) in samples of cerebrospinal fluid.

### STATE OF THE PRIOR ART

Cerebral and cerebro-vascular accumulation of a 4-kDa peptide termed beta-Amiloyd (Abeta) or beta amyloid protein, is widely accepted as the key neuropathological event in Alzheimer disease (AD). AD main features are in fact amyloid plaques, mainly formed by Abeta aggregates (Abeta40 and Abeta42), and Abeta deposition at vessel level, also referred to as Cerebral Amyloid Angiopathy, deriving by proteolysis of the amyloid precursor protein (APP) by beta- and gamma-secretase.

Various mechanisms seem to be accountable for the formation of these accumulations, among which a malfunctioning of toxic aggregate clearance systems or a malfunctioning of cell defence systems at cerebral level. Therapeutic approaches defined as "Amyloid-modifying therapies (Abeta-DMT)", and in particular those founded on active and passive immunisation, are based precisely on a possible restoration and potentiation of these mechanisms.

Strong stimuli in the use of these therapies ensue from previous evidences in transgenic animal models, in which it has been demonstrated that immunisation is capable of preventing, and sometimes of reducing, the presence of beta amyloid plaques. Initially, active immunisation approaches were undertaken on patients, by administering beta amyloid protein and thereby fostering a spontaneous endogenous antibody production directed against the injected antigen. Due to the impossibility of monitoring antibody production in the patient by this strategy, besides the fact that the beta amyloid protein is ubiquitously distributed in the body and highly dishomogeneous from one patient to another in terms of deposition and circulating levels, these clinical trials were discontinued in phase 1 or 2, due to occurrence of episodes of acute meningoencephalitis.

Thanks to the remarkable progresses in terms of pharmaceutical preparations and thanks to the remarkable advancement of scientific knowledge in the field of the production and characterization of antibodies anti-beta amyloid protein, clinical-pharmaceutical research today is almost exclusively engaged in clinical trials based on the injection of humanized antibodies by systemic route. This immunisation technique, termed "passive", entails the advantage of being capable of better controlling the circulating antibody titre, and of hypothetically interrupting the pharmacological activity in case of need.

In fact, despite the greater safety of these therapeutic strategies some collateral risks do persist, among which the most relevant one is the development of vasogenic edema (VE).

In animal models, it has been proved that these adverse phenomena, such as vasogenic oedema and neuroinflammatory response, are strictly related to the therapeutic dose of the antibodies administered. Actually, animals treated with low dosages showed no occurrence of the above-mentioned adverse effects. Moreover, of particular interest is the fact that, by decreasing the therapeutic dose in animals that were beginning to show signs of restlessness, the adverse effects themselves could be reverted.

Hence, it is essential to be able to dose the levels of antibodies present at cerebral level, in the cerebrospinal fluid (CSF), i.e. the levels of antibodies which actually succeed to reach the cerebral district and therefore to have a central pharmacological effect. It follows that, in order to be able to effectively evaluate a rise of such levels in the CSF, it is essential to avail oneself of a dosage highly sensitive and capable of detecting also the presence of the autoantibodies spontaneously present in basal conditions, i.e. prior to the treatment. Moreover, thanks to very recent evidences deriving from clinical studies and pharmacological trials, it is known that knowing the exact situation of cerebro-vascular impairment in subjects starting the therapy is fundamental, since subjects with proneness to endothelial damage or carriers of cerebro-vascular risk factors, such as the presence of APOE4 haplotype, can incur a 3% to 5% higher percentage of developing VE, and therefore even death.

Common techniques enabling to evaluate beta amyloid deposition and the presence of cerebral endothelial damage are imaging techniques with PET and MRI. However, these techniques are very costly, not yet fully validated and acknowledged in clinical routine, require highly qualified staff and specific instruments, are not indicative of a real biological response and therefore are used only in exceptional cases and accompanied by a chemical-physical investigation. Moreover, it has to be pointed out that these techniques show nothing with regard to the concentration of antibodies spontaneously present in the CSF (autoantibodies), or to how much they have increased following the treatment or, again, to their connection with cerebro-vascular impairment.

Recently, however, such evidences were revisited and acquired even greater relevance after the *Alzheimer Association Research Workgroup* defined the concept of ARIA, i.e. of "amyloid-related imaging abnormalities" represented by the development of vasogenic oedema and/or sulcal effusion (ARIA-E) using FLAIR sequences, and signals of hemosiderin deposition (ARIA H) at T2* sequences in echo gradient (T2*-GRE), in Alzheimer patients treated with the monoclonal antibody bapineuzumab, and subsequently confirmed also in other treatments, like e.g. with semagacestat and solanezumab.

This proves even more interesting given the many similarities shared between the ARIA present in these AD patients and a very specific subclass of patients suffering from cerebral amyloid angiopathy-related inflammation (CAA-ri), in which a specific autoimmune mechanism by autoantibodies against the beta amyloid protein seems to underlie the etiopathogenetic mechanism and ARIA appearance in these subjects. However, it has to be stressed that to date the pathogenetic mechanisms underlying ARIA, both in AD and CAA-ri, are still largely unknown. To date, a firm diagnosis of CAA-ri, essential for a correct treatment of acute phases, is possible only upon cerebral biopsy, i.e. by an investigation very invasive and risky for the patient, given the increased proneness to bleeding characteristic of the pathology, and an accurate NMR investigation. Should the autoantibodies turn out to be the actual mechanism characterizing the acute phase of the disease, and directly correlated to their concentration in the CSF, obviously we might therefore avail ourselves of a much less risky and invasive alternative diagnostic tool for the diagnosis of CAA-ri.

As for the dosage of antibodies directed against beta amyloid protein, to date there is all but one study in the literature (Du Y *et al.*)*,* published in 2001 on *Neurology* journal, describing a method for plasma dosage of autoantibodies (i.e., those naturally occurring in the plasma) directed against Abeta40 protein. This method is exclusively based on a mere ELISA methodology and represents all but the only method that may be found in the literature for the dosage of the antibody titre against Abeta protein in the plasma of patients suffering from AD and from Down syndrome.

To date, however, net evidences indicating the exact distribution of the plasma antibody titre in AD subjects compared to healthy population remain few and hazy. In fact, the plasma titre of autoantibodies anti-Abeta protein seems to be decreased according to some (Du Y et al., Weksler ME et al., Song MS et al.), unvaried according to others (Hyman BT et al., Baril L et al.), or even increased according to still others (Nath A et al.).

This is certainly true and highlighted as regards autoantibodies dosage at CSF level (Du Y et al.). In fact, despite in plasma the antibody titre is sufficiently high to be dosed relatively straightforwardly, the method described by *Du Y et* al is nearly useless for dosage in the CSF, in which autoantibodies are present at an extremely low concentration, and therefore non-dosable with sufficient sensitivity and reliability by the ELISA methodology used by Du Y et al. In the discussion section of the two titles by Du Y et *al*., the same authors specify that they could isolate and purify the antibodies only from plasma and not from CSF, as a too large amount would be required.

In Patent application WO2011009435 an ELISA method for the detection of autoantibodies in samples of plasma and also of CSF is described, though reporting no data highlighting the greater sensitivity of this method compared to that described in the prior art for dosage in the CSF.

The international patent application WO2010128139 discloses a method of diagnosing Alzheimer's disease (AD) in a subject, comprising: (a) determining, in a sample derived from a body fluid of said subject, the amount of antibodies capable of binding the specific isoform pGLU of the amyloid αβ peptide; and (b) comparing the amount of said antibodies to a reference amount; wherein the amount of said antibodies compared to the reference amount is indicative that the subject is affected by AD

As for the dosage of antibodies anti-beta amyloid protein in CSF samples, the main problem remains therefore the very low concentration of autoantibodies present, values so low that it is demonstrable that all are encompassed between zero, i.e. blank, and the first point of the calibration curve standard, i.e. too easily mistaken for background noise. Relevant however is the evidence that, despite a massive commitment of the pharmaceutical industry and of research in general in identifying ever more effective and safe therapeutic antibodies, very little or nothing is known about the concentration of autoantibodies spontaneously occurring at cerebral level in patients suffering from AD and CAA in general. The rationale pursued to date has been that of assuming a decreased antibody presence in AD patients; therefore, the focus has only been on exogenous administration, by systemic route, of therapeutic antibodies capable of demonstrating a slowing down of the pathology. To date, such evidences are supported only by the demonstration that in animal AD models such therapies actually decrease Abeta deposition at cerebral level. However, due to the technical-experimental difficulty in dosing autoantibodies, present in a very low concentration at CSF level, it is not yet known exactly whether AD subjects actually exhibit a decreased antibody titre prior to therapy and, above all, it is not known how many therapeutic antibodies actually succeed at passing the blood-brain barrier and therefore at having a therapeutic effect in the cerebral district, or, again, which be their concentration trend during therapeutic administration, or even more importantly, which be their concentration during the appearance of the radiological abnormalities (ARIA). The same applies to CSF concentration of antibodies naturally produced during the acute phase of CAA-ri and after immunosuppressive therapy, following a clinical-radiological resolution of symptoms in the remission phase.

Hence, the problem of providing a method for dosing with a high sensitivity the concentration of antibodies anti-beta amyloid protein present in CSF samples is highly felt.

### SUMMARY OF THE INVENTION

The method of the present invention is based on the selection and development of a step of concentrating the antibodies anti-beta amyloid protein originally present in a sample of cerebrospinal fluid and on the subsequent detection of the antibodies thus concentrated in an immunoenzyme assay or a radioimmunoassay.

The inventors have discovered that with magnetic micro beads, suitably coated with a macromolecule capable of binding antibodies directed against beta amyloid protein, a concentrated sample is obtained from which it is possible to quantitatively detect the concentration of antibodies present in the starting sample, even when this concentration is extremely low. The Inventors have demonstrated that the step of concentrating on micro beads determines an increase in the sensitivity of the immunoenzyme assay or radioimmunoassay utilized for determining antibodies concentration. The micro beads, by being magnetic, can be easily removed with a magnet, therefore leaving no dead volumes that might cause a sample loss.

Also particularly effective steps of the method described herein have been developed, which will be described in depth in the detailed description of the invention.

By the method of the present invention for the first time in the state of the art, the actual concentration of antibodies present in the CSF of subjects under treatment with active and/or passive immunisation therapies, and of Abeta-DMT in general could be dosed, thereby enabling a more effective monitoring of the therapy and to prevent ARIA appearance in the patient; yet, also antibody concentration spontaneously occurring in the CSF of patients candidate for a possible therapy could be dosed, as a pre-therapeutic and/or diagnostic screening, in line with an improved therapeutic customization.

A first object of the present invention is an *in vitro* method for the detection of antibodies anti-beta amyloid protein in a sample of cerebrospinal fluid according to claim 1

A further object of the present invention is an *in vitro* method for monitoring an active and/or passive immunisation treatment of the Alzheimer disease, from a CSF sample of a patient suffering therefrom, comprising the steps of anyone of claims 1 to 7.

Yet another object of the present invention is an *in vitro* method for the diagnosis of cerebral amyloid angiopathy (CAA) and of CAA-related inflammation, autoimmunity and vasogenic oedema (CAA-ri) from a CSF sample of a patient comprising the steps of anyone of claims 1 to 7.

Yet another object of the present invention is an *in vitro* method for the diagnosis or the therapeutic monitoring of radiological abnormalities (ARIA) from a CSF sample of a patient comprising the steps of the method according to anyone of claims 1 to 7 and a further step of comparing the determined concentration of antibodies anti-beta amyloid protein with a standard reference value.

### BRIEF DESCRIPTION OF THE FIGURES

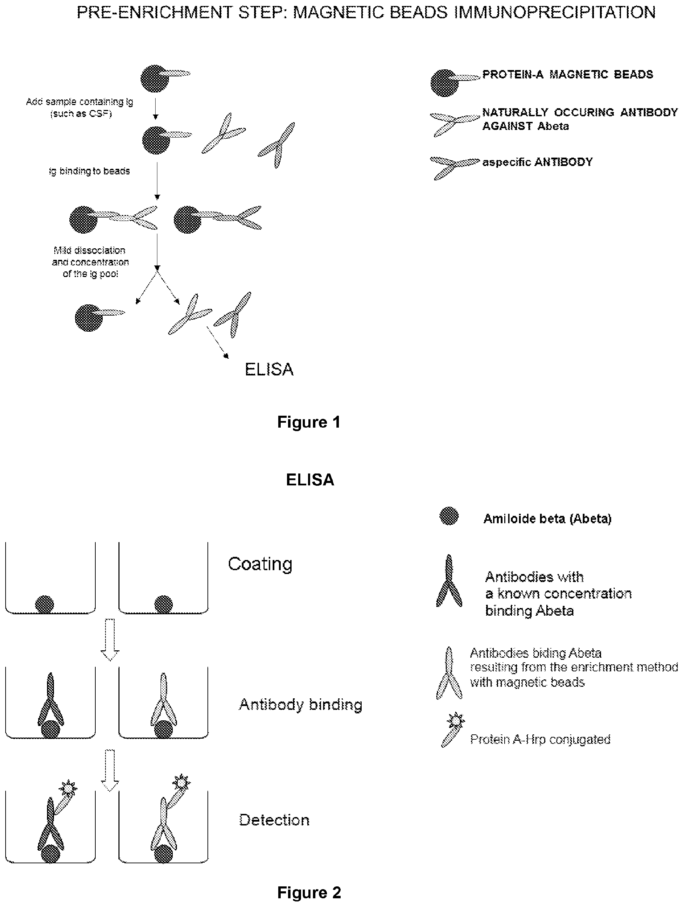

Figure 1. Schematic representation of step a) of concentrating the sample according to an embodiment of the method of the present invention.
Figure 2. Schematic representation of step b) of analysing the sample by immunoenzyme assay according to an embodiment of the method of the present invention.

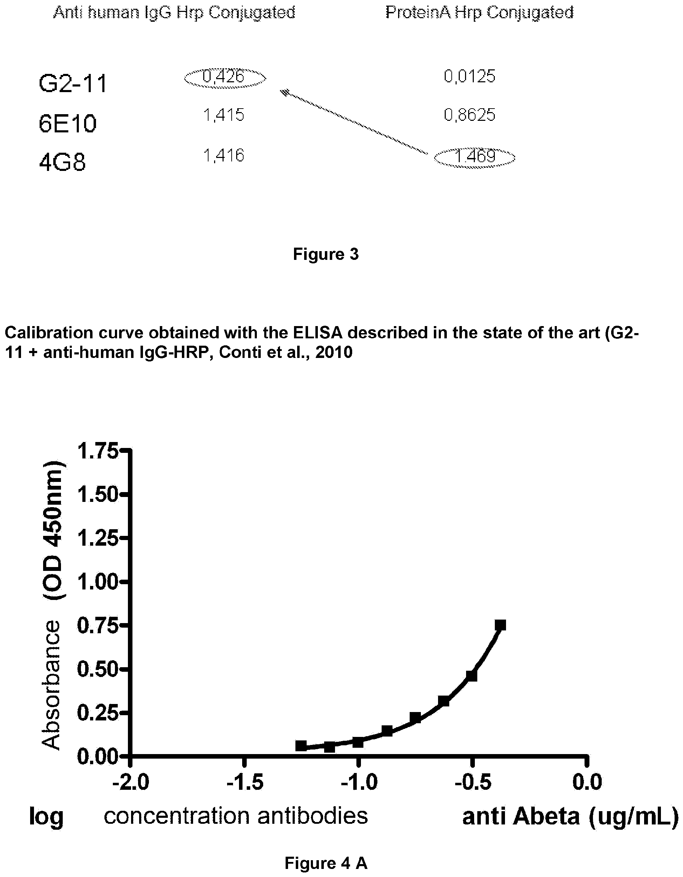

Figure 3. Reports a comparison between Absorbance values (OD) found by analysing three commercially available different antibody clones against Abeta (clone G2-11, distributed by The Genetics Company; clones 6E10 and 4G8, distributed by Covance), at the same known concentration of 0.25 µg/ml, without performing any concentration with magnetic micro beads. In the left-side column, OD values for the three commercial antibodies tested by ELISA procedure described in the state of the art are reported. The right-side column reports the OD values obtainable by our new ELISA method. As secondary antibody an Hrp-conjugated anti-human IgG produced in goat, (Anti-Human IgG (whole molecule)-Peroxidase antibody produced in goat, SIGMA #A8667), or Hrp-conjugated protein A (Protein A, conjugated to Hrp, Millipore #18-160) was used.
Figure 4a. Calibration curve. Results obtained with the ELISA procedure described in the state of the art (standards used without pre-enrichment), utilizing, unlike what taught in the state of the art, as antibody for the calibration curve the antibody G2-11, and as secondary antibody an Hrp-conjugated anti-human IgG, produced in goat (Conti et al 2010). It can be seen that the calibration curve with the method in the state of the art was not sufficiently sensitive and accurate for the dosage of CSF samples, both for untreated samples and for CSF concentrated with micro beads according to the method described by the Inventors. In fact, it can be observed that the CSF samples demonstrate a very low OD value, falling within the points of the calibration curve comprised between zero (blank) and the first point of the curve, i.e. totally non-measurable in practical terms.

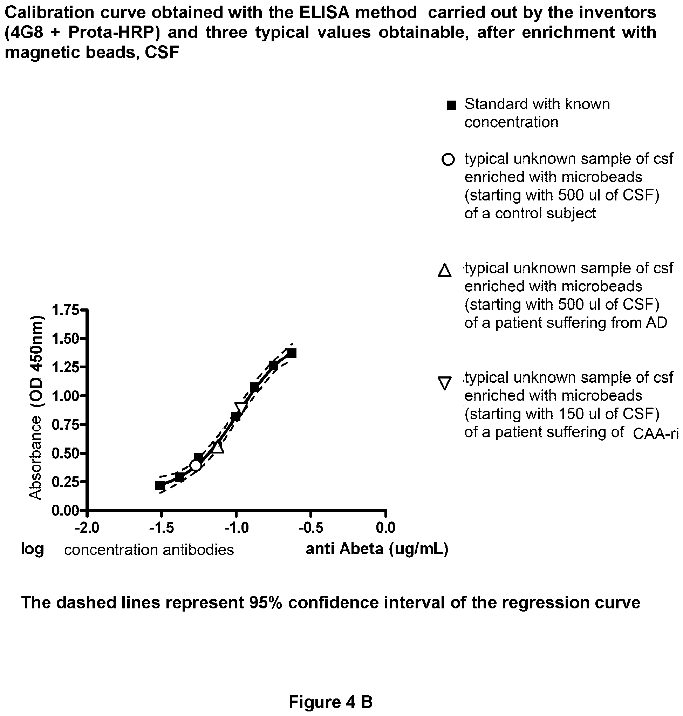

Figure 4b. Calibration curve. Results obtained utilizing as antibody for the calibration curve the antibody 4G8 and as secondary antibody the Hrp-conjugated Protein A (curve standards were always utilized without pre-enrichment) but with a particular selection and combination of reagents for the detection implemented by the Inventors and not described in the state of the art. The figure shows how the selection of clone 4G8 + protA-HRP has a greater sensitivity compared to the method described in the state of the art, above all for those concentration values of the standards very low and that had proved unusable in the state of the art.
   Moreover, typical values obtainable by analysing CSF samples obtained from patients suffering from AD, CAA-ri, and controls are reported. It may be observed that for all 3 cases shown the OD are perfectly comprised within the linearity range of the standard curve.

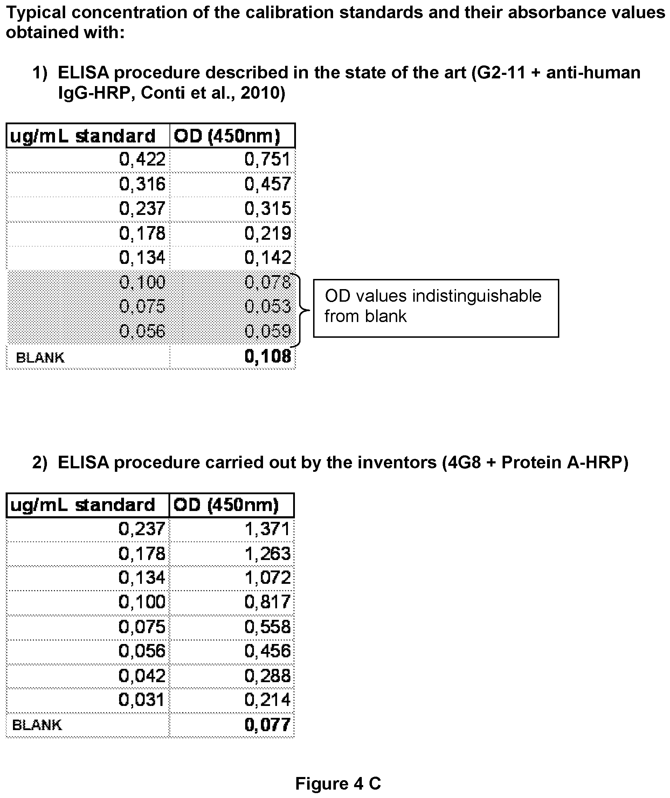

Figure 4c. Typical concentration of calibration standards and related absorbance values (OD). Typical results are shown, obtained with:
   1) ELISA procedure described in the state of the art (G2-11 + anti-human IgG-HRP, Conti et al., 2010)
   2) ELISA procedure implemented by the Inventors (4G8 + protA-HRP)

   As usual, standards undergo no enrichment process with magnetic beads.
   The Figure shows how, for a typical calibration curve, our novel ELISA method proves to be ultra-sensitive in the detection range expected for the quantification of antibodies anti-Abeta in the CSF (between 0.14 and 0.04ug/mL). It is moreover shown how, compared to the old ELISA method described in the state of the art with antibody G2-11, at the same concentration of 0.134 ug/mL, the OD value detectable with our new ELISA method is 1.072, compared to the 0.142 detectable with the ELISA method described in the state of the art. This demonstrates how our new method is about 10 times more sensitive to such concentration values. Moreover, it is observed that for all standards with a concentration lower than 0.134 ug/mL the prior method was unable to discriminate any unknown value from the blank.

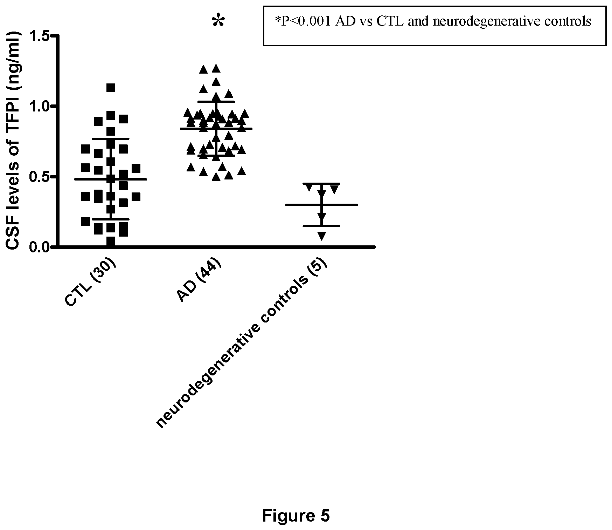

Figure 5. TFPI dosage in samples of CSF from patients suffering from Alzheimer disease (AD) compared with healthy controls (CTL) and other patients suffering from neurodegenerative pathology without vascular component (neurodegenerative controls). Results were obtained by modifying the experimental procedure described in Quantikine Human TFPI ELISA KIT - R&D Systems. In particular, the protocol provided by the manufacturing company was assayed and modified in order to utilize the KIT also for dosage in the CSF (the description of the method used is in the EXAMPLES section, Example 7). As may be noted in the figure, TFPI values in the CSF of 44 AD patients proved to be significantly increased compared to a group of 30 healthy controls and 5 subjects suffering from a neurodegenerative pathology such as Amyotrophic Lateral Sclerosis, a pathology in which no association with cerebro-vascular damage is reported.

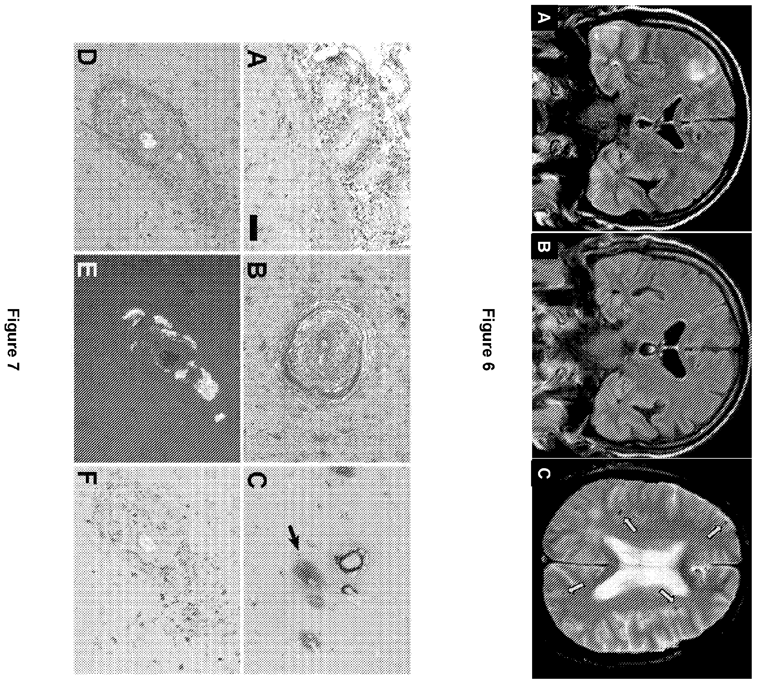

Figure 6. Nuclear Magnetic Resonance frame of a CAA-ri patient (case 6); brain MRI coronal FLAIR shows bilateral lesions of subcortical white matter during the acute phase of the disease (A), reducing after 3 months with spontaneous remission of symptoms (B); indicates cerebral MR with axial sessions of T2-star sequences or of gradient echo (C) detects the presence of multiple and bilateral microbleeds in the white matter (arrows). No contrast enhancement was present after Gadolinium injection (data not shown).
Figure 7. Neuropathological evidence at bioptic analysis of right temporal lobe, case #6. H&E (A,D) and Masson trichrome (B) stains show a thickening of the leptomeningeal and parenchymal vasal bed, with lumen reduction. Moreover, lymphomonocytic infiltrations were highlighted in the perivascular space and in the vasal bed thickening (CD45 immunostain, F). The material emerged from the vasal bed proved intensely immunoreactive for protein Aβ (monoclonal antibody 4G8, C) with optical properties and stain typical of the amyloid protein (yellow fluorescence after Thioflavine S treatment, E). Anti-Aβ antibodies moreover detected numerous deposits diffused in the cortical neuropilum (4G8, arrow, C), in the absence of neurofibrillary changes (data not shown). In addition, severe astrogliosis and microglial activation were found in the cerebral cortex and in the subcortical white substance.
   Bar represented in A = 50 µm. All other figures have the same degree of magnification. D, E and F were performed in adjacent sections.

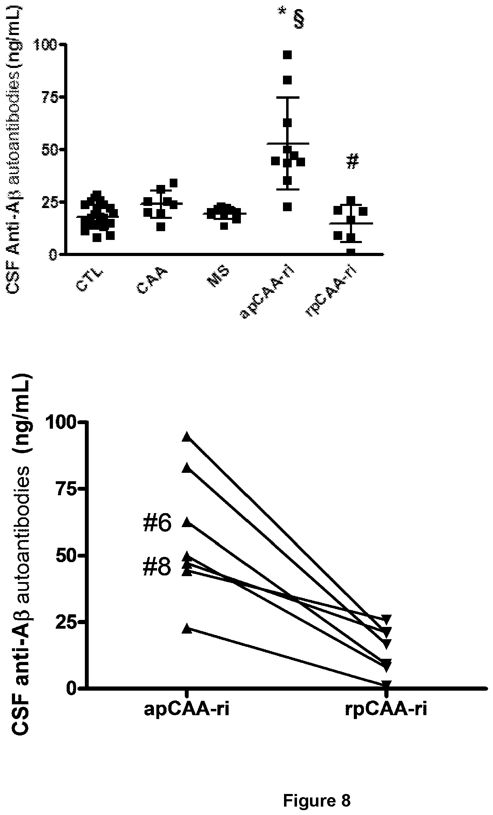

Figure 8. Concentration of anti-Aβ antibodies in the CSF
   **A)** Concentration of anti-Aβ autoantibodies in the CSF of healthy subjects (CTL, n=25), cerebral amyloid angiopathy (CAA, n=8), multiple sclerosis (MS, n= 14), CAA-related inflammation during the acute phase (apCAA-ri, n=10) and CAA-ri in the remission phase (rpCAA-ri, n=7). * p <0.0001 apCAA-ri vs CTL and MS. § p <0.0025 apCAA-ri vs CAA. # p <0.0006 apCAA-ri vs rpCAA-ri. Values are expressed as mean ± standard deviation.
   **B)** Case-by-case representation of the trend of the antibodies before and after the phase of remission of symptoms in apCAA-ri and rpCAA-ri patients (p=0.0009). Cases 6 and 8 are shown in the figure and represent the 2 patients in which a spontaneous remission of symptoms was observed without any pharmacological treatment.

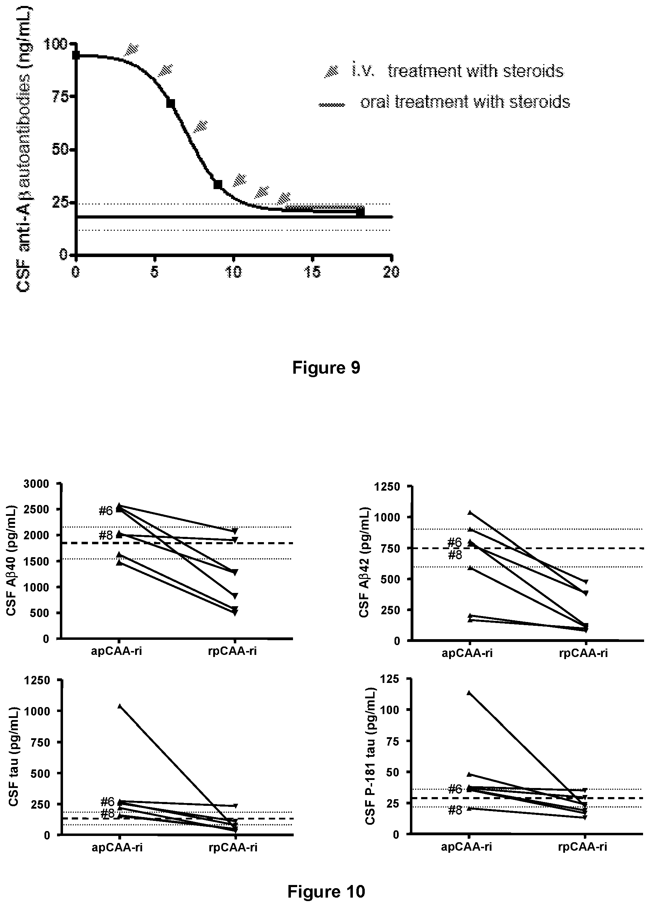

Figure 9. Progressive reduction over time of anti-Aβ antibodies in the CSF of a CAA-ri patient (case 5) after each intravenous injection (arrows) and during oral treatment (line) with steroids, until reaching average values that can be found in healthy controls (dotted lines represent the average value and 95% of the upper and lower mean value that can be found in healthy control subjects).
Figure 10. Aβ40, Aβ42, tau and P-181 tau concentration and related progression of levels in the CSF of patients suffering from cerebral amyloid angiopathy-related inflammation (CAA-ri, n=7) during the acute phase (apCAA-ri) and after the phase of remission of symptoms (rpCAA-ri). **A)** Aβ40 (p=0,009, apCAA-ri vs rpCAA-ri, p=0,032 rpCAA-ri vs CTL), **B)** Aβ42 (p=0.015, apCAA-ri vs rpCAA-ri; p=0.002, rpCAA-ri vs CTL), **C)** tau (p=0.004, apCAA-ri vs rpCAA-ri; p=0.012, apCAA-ri vs CTL) and **D)** P-181 tau (p=0.007, apCAA-ri vs rpCAA-ri; p=0.044, apCAA-ri vs CTL). Cases 6 and 8 are indicated in the figure and represent the 2 patients in which a spontaneous remission of the symptoms was observed without any pharmacological treatment (broken lines represent the mean value and the 95% of the upper and lower mean value that can be found in healthy control subjects).

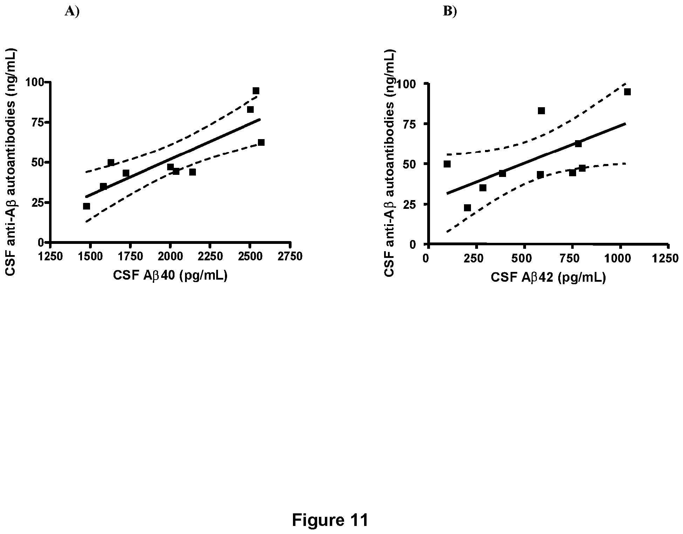

Figure 11. Demonstration of the existence of a strong correlation between anti-Aβ antibodies and liquor (CSF) values of Aβ40 (**A,** Pearson r=0.85, p=0.002) and Aβ42 (**B**, Pearson r=0.65, p=0.042) in the total population of 10 apCAA-ri. Solid and broken lines represent the regression line and the 95% confidence interval, respectively).

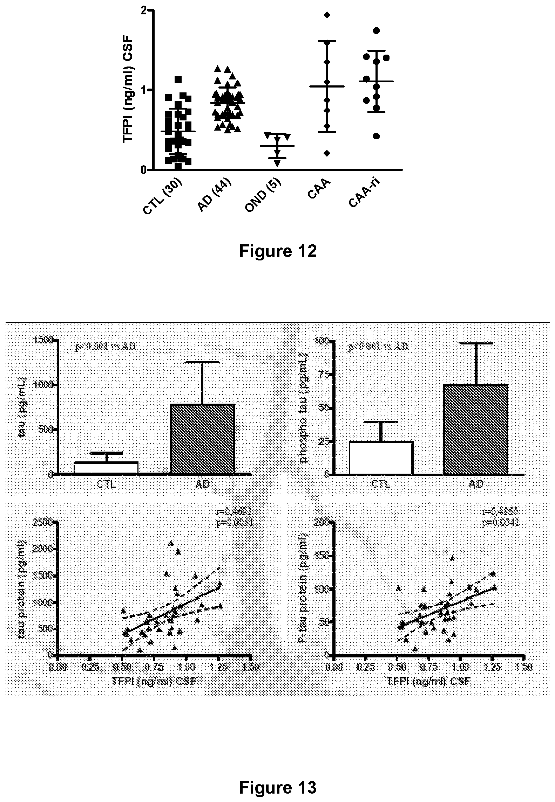

Figure 12. CSF values of TFPI. Legend: TFPI concentration in the CSF of healthy subjects, cerebral amyloid angiopathy (CAA, n=8), Amyotrophic Lateral Sclerosis (OND) and CAA-related inflammation (CAA-ri, n=10). * p <0.0001 CTL vs CAA-ri, CAA and AD. Values are expressed as mean ± standard deviation.
Figure 13. CSF values of tau, P-181 tau and related correlation analysis with the TFPI values. Legend: Values of tau and P-181 tau in the CSF of AD patients and healthy controls (top boxes). Bottom boxes report the correlations emerged between the CSF concentration of TFPI and of tau and P-181 tau (p<0.0051, r = 0.47; p<0.0041, r = 0.49, respectively).

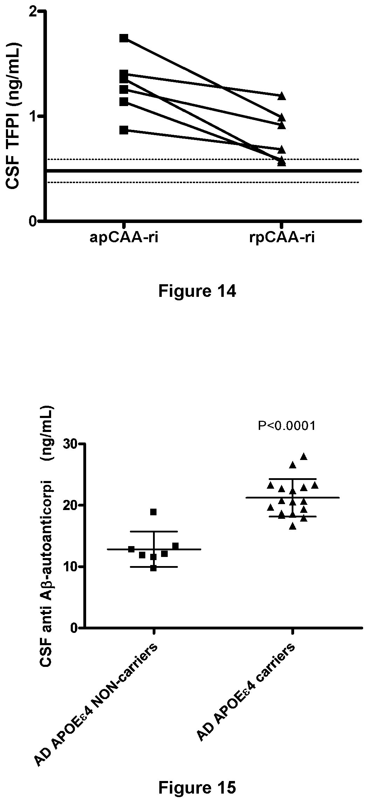

Figure 14. Trend of TFPI values in the CSF of patients during apCAA-ri and rpCAA-ri. Legend: case-by-case representation of the trend of CSF TFPI before and after the phase of remission of symptoms in apCAA-ri and rpCAA-ri patients (p=0.01). (Dotted lines represent the mean value and the 95% of the upper and lower mean value that can be found in healthy control subjects).
Figure 15. As shown in the Figure, levels of anti-Aβ antibodies present in the CSF of Alzheimer patients were evaluated by the method of the present invention, observing that patients carriers of the genetic risk factor ApoE epsilon4 (APOE4 carriers) have a greater physiological concentration of autoantibodies (12.84 ng/mL ± 21.23) compared to NON-APOE4 carrier patients (2.874 ng/mL ±3.063; p<0.001), in support of a greater predisposition by APOE4 carriers to develop ARIA, both spontaneously and following Abeta-DMT administration.

### DETAILED DESCRIPTION OF THE INVENTION

The present invention provides for the first time a high-sensitivity method intended for the detection of antibodies anti-beta amyloid protein in a sample of cerebrospinal fluid.

To the ends of the present description, by "antibody against beta amyloid protein" it is meant any antibody that be capable of selectively binding any one portion of the beta amyloid protein, in the literature also termed Abeta, or isoforms thereof, like, e.g., Abeta40, Abeta42, Abeta43, or any possible truncated, racemic or isomer form thereof (e.g., Abeta11-42, Abeta17-42, Abeta9-42, Abeta1-28, Abeta25-35, Abeta10-20, or more generally AbetaX-40 or AbetaX-42) or their respective various monomer, dimer or oligomer, soluble, insoluble, fibrillar or protofibrillar forms.

Detected antibodies could be autoantibodies, i.e. antibodies produced by the immune system of the patient from which the cerebrospinal fluid has been collected, or antibodies not produced by the immune system of the patient, like e.g. antibodies for therapeutic use administered to the patient (like, e.g.: Solanezumab, Ponezumab, Bapineuzumab, Gantenerumab, BAN2401, BIIB037, intravenously administered immunoglobulins (IVIG) such as Gammagard, Octagam, Newgam). Therefore, detected antibodies could be both endogenous and exogenous.

The sample of cerebrospinal fluid (also referred to as liquor, cerebrospinal fluid, rachidial-spinal fluid, cephalorachidian fluid (liquid), or with the acronym CSF) to be analysed could be obtained according to the various techniques commonly used for CSF collection. The method according to the invention, as already anticipated, comprises two steps a) and b), respectively of concentrating the quantity of said antibodies of the CSF sample with magnetic micro beads coated with macromolecules capable of binding said antibodies, and of analysing the thus concentrated sample obtained by immunoenzyme assay or by radioimmunoassay.

### a) Step of concentrating with magnetic micro beads

The magnetic micro beads suitable for the method described herein could be uniform particles, typically of from 0.5 to 500 µm in diameter, whose surface is coated with macromolecules capable of binding antibodies directed against beta amyloid protein, like e.g. Protein G, protein A, biotin, streptavidin, any one portion of the Abeta protein and/or isoforms thereof, like e.g. Abeta40, Abeta42, Abeta43, or any possible truncated, racemic or isomer form thereof (e.g., Abeta11-42, Abeta17-42, Abeta9-42, Abeta1-28, Abeta25-35, Abeta10-20, or more generally AbetaX-40 or AbetaX-42) or their respective various monomer, dimer or oligomer, soluble or insoluble, fibrillar or protofibrillar forms.

The coating of the micro beads with the macromolecules could be carried out by adsorption or conjugation, as known to the technician in the field.

Types of suitable micro beads are, e.g., Invitrogen Dynal AS Dynabeads Protein G, protein A or proteins A/G, MagSi-protein A and/or protein G beads, Millipore Magnetic beads, Miltenyi Biotec MACS® Technology beads, or any other type of magnetic beads with the same features described above and capable of being used both with manual procedures and by all possible robotized and automated platforms (e.g., produced and/or marketed by Beckmann®, Tecan®, IDS®, Xyril®, Stratek®, Hamilton®, Roche®, PerkinElmer®, etc.).

In an embodiment of the invention, as beads also agarose A/G beads or sepharose beads could be used, which however cannot be easily usable on automated platforms and that might give a lesser concentration yield with possible loss of some part of the sample.

In an embodiment, a volume of at least 200 µl, e.g. 400 or 500 µl of sample of CSF to be analysed is adsorbed on the micro beads, for instance by incubating under stirring in a same test tube the sample and a suitable amount of micro beads (e.g., 75ul) for a period of time suitable to bind the antibodies present in the sample to the micro beads, (e.g., 30, 40, 50, 60 minutes).

After the step of adsorption, the test tube containing the sample and the micro beads is placed near a magnet so as to be able to discard the supernatant with conventional techniques, as by pipette or suction, without losing the substrate of interest, or by automated and robotized platforms. The supernatant could be stored and utilized as control material in the subsequent immunoenzyme assay or radioimmunoassay.

Generally, the steps of adsorption of the sample on the micro beads and of discarding the supernatant could be preceded and/or followed by one or more washings of the micro beads with a washing buffer, like e.g. PBS + Tween according to conventional techniques.

Upon discarding the supernatant and, when desired, having suitably washed the micro beads, bound antibodies could be eluted from the micro beads with a suitable elution buffer, for instance when it is necessary to free suitable epitopes for subsequent antibody detection.

In case the micro beads be coated with protein A or protein G, the elution buffer could be made of a volume comprised between 30 and 50 µl of 0.1M citrate at pH 3.2. Upon eluting the sample, the acidic pH will be readily neutralized with a concentrated basic buffer (e.g. 1M tris base, pH 9). Addition of the neutralization buffer is important to avoid that the acidic pH used in the elution phase may denaturate the antibodies directed against beta amyloid protein of which the concentration is to be determined, or create artefacts thereof which might generate false positives during the subsequent immunoenzyme assay.

In an alternative embodiment, wherein the micro beads are coated with beta amyloid protein and/or isoforms thereof, like e.g. Abeta40, Abeta42, Abeta43, or any possible truncated, racemic or isomer form thereof (e.g., Abeta11-42, Abeta17-42, Abeta9-42, Abetal-28, Abeta25-35, Abeta10-20, or more generally AbetaX-40 or AbetaX-42) or their respective various monomer, dimer or oligomer, soluble or insoluble, fibrillar or protofibrillar forms) and therefore will specifically bind only the antibodies anti-beta amyloid protein contained in the sample of CSF to be analysed, the micro beads could be utilized directly in the subsequent step of analysis with immunoenzyme assay or radioimmunoassay, avoiding the elution step and therefore with a quicker, more sensitive and less expensive method.

The sample concentrated with the magnetic micro beads (eluted or not eluted by the same) will subsequently be subjected to a second step of b) analysing by immunoenzyme assay or by radioimmunoassay.

### Analysing by immunoenzyme assay

In the embodiment in which at step a) the elution as described above is performed, said immunoenzyme assay provides the following steps:
*I) providing a solid phase coated with beta amyloid protein;*
   said solid phase could be a solid phase conventionally used in immunoenzyme assays or in immunoassays, like e.g. a microplate in which the wells are coated with the beta amyloid protein as defined above, properly blocked to avoid background noise with methods them also conventional, like by treatment with a phosphate buffer solution (pH 7.4) with 1% bovine albumin, 5% serum and 0.05% Tween.
   The incubation time of the blocking solution will be easily identifiable by the average technician in the field and could, for instance, be of about 30, 40, 50, 60, 90 minutes. To remove the unbound material, the solid phase could be rinsed once or more with suitable conventional washing buffers, like PBS + 0.05% Tween.
*II) incubating said solid phase with the concentrated material obtained from step a);*
   in step II) the sample concentrated with the magnetic micro beads will be added to the solid phase, thus allowing the binding of the antibodies to the beta amyloid protein and/or isoforms thereof like e.g. Abeta40, Abeta42, Abeta43, or any possible truncated, racemic or isomer form thereof (for instance Abeta11-42, Abeta17-42, Abeta9-42, Abeta1-28, Abeta25-35, Abeta10-20, or more generally AbetaX-40 or AbetaX-42) or their respective various monomer, dimer or oligomer, soluble or insoluble, fibrillar or protofibrillar forms) on the solid phase. The incubation time will be easily identifiable by the average technician in the field and could, e.g., be of about 30, 40, 50, 60 minutes, up to an incubation also overnight. To remove the unbound material, the solid phase could be rinsed once or more with suitable conventional washing buffers, like PBS + 0.05% Tween.
*III) incubating the solid phase obtained in step II) with a protein conjugated to an enzyme, or to a radioactive label capable of binding human antibodies, followed by rinsing to remove said unbound conjugated protein;*
   in case an immunoenzyme assay is carried out, according to the present invention, the protein conjugated to an enzyme could be, e.g., protein A conjugated to horseradish peroxidase (**HRP**) enzyme, protein G conjugated to Hrp enzyme, antibodies conjugated to Hrp enzyme or to alkaline phosphatase (**ALP**) or to beta galactosidase, or to a fluoresceinated molecule or to a luminescent molecule (e.g., luminol) capable of selectively binding the Igs of any species; moreover, any reagent capable of binding the Igs and yielding a signal detectable in the visible UV spectrum or under fluorescence, not only antibodies and conjugated proteins but also suitably functionalized nanoparticles or liposomes, could be utilized.
   In case instead a radioimmunoassay is used, a protein conjugated to a radioactive label , e.g. G protein conjugated to I-125, I-131 or I-99, A conjugated to I-125, I-131 or I-99, anti-IgG antibody conjugated to I-125, I-131 or I-99, could be utilized.
   Each specific antibody present in the sample will thus bind to the radioactive agent and, during the second stage of the incubation, the antigen-antibody complexes may be precipitated thanks to a precipitating agent (if in solution) or washed with a suitable washing buffer.
*IV) detecting the signal of said conjugated protein.*

In case of an immunoenzyme assay, after washing to remove the excess of unbound conjugated protein, a solution of suitable substrate, selected according to conventional procedures on the basis of the enzyme conjugated to the protein, will be added to the well in order to allow development of the signal that could then be quantified according to standard techniques. The signal could for instance be quantified by absorbance or fluorescence reading with a suitable tool, depending on the type of enzyme and of related substrate used. Signal strength is proportional to the quantity of antibodies present in the sample to be analysed.

In case of a radioimmunoassay, after centrifuging and supernatant settling, or after the step of washing in case of RIA or IRMA assay with antibodies adhering to the solid phase, precipitate radioactivity is counted through a gamma counter. Radioactivity strength will be proportional to the concentration of the specific antibody present in the sample deriving from the patient. Antibody concentration could then be quantitatively measured through a calibration curve.

In an embodiment, the conjugated protein could be protein A conjugated to horseradish peroxidase (A-HRP). In the immunoenzyme assays described in the state of the art, two different secondary antibodies are used: one for samples having a known titer (for instance those used for elaborating the standard curve, e.g. anti-mouse IgG antibody) and one for unknown samples (anti-human IgG antibody). The use of HRP-conjugated protein A in the method of the present invention entails the further advantage of having the same detection system both for samples having a known titre, usually of murine origin, and for the unknown sample to be analysed, obviating to differences, in terms of sensitivity, given by species-specificity of antibodies and therefore making the method more versatile, as usable on samples of human origin as well as of murine origin, of rat or of other species. In fact, the HRP-conjugated protein A binds in a highly specific way both to human and mouse IgG, obviating the problem of having two different secondary antibodies, which therefore might yield different sensitivity and species-specificity, hence making the result less reliable during the extrapolation of the unknown sample concentration from the standard curve. Moreover, the selection of protein A-HRP enables, in step IV) of detecting the signal, to use a fluorescence-emitting substrate, and therefore with a greater sensitivity compared to absorbance-emitting substrates, in case it becomes necessary; for instance, by utilizing ultra RED Amplex technology which amplifies the HRP signal.

The invention also relates to an *in vitro* method for the quantification of antibodies anti-beta amyloid protein in a sample of CSF.

Methods enabling a quantification of these antibodies in the CSF are not known in the literature; such a quantification obviously is of fundamental relevance both to diagnostic and prognostic ends, as well as for monitoring the course of therapies for the treatment of Alzheimer disease and of CAA.

Object of the present invention is also a high-sensitivity *in vitro* method for the quantification of antibodies anti-beta amyloid protein in a sample of CSF, comprising the following steps:
a) concentrating the quantity of said autoantibodies of said sample with magnetic micro beads coated with macromolecules capable of binding said autoantibodies;
b) analysing the concentrated sample obtained in step a) by immunoenzyme assay or by radioimmunoassay.
c) analysing a sample comprising antibodies anti-beta amyloid protein having a known titre with the same assay used in step b) and elaborating the related calibration curve.

Steps a) and b) could be carried out according to any one of the above-described embodiments.

According to the method of the invention, in step c) an antibody having a known titre, capable of selectively binding any one portion of the beta amyloid protein, will be analysed according to the same procedure used in step b) in order to elaborate a calibration curve according to conventional techniques.

For instance, starting from a more concentrated solution of antibody having a known titre, the various points of the calibration curve related to successive dilutions could be prepared. Said antibody could be any one monoclonal, polyclonal antibody, immunologically active antibody fragments such as Fab, Fab2, scFv having a titre known and specific for the beta amyloid protein, therefore directed against a specific epitope of the beta amyloid protein.

In an embodiment, said antibody or a fragment thereof could be directed against an epitope comprised in the N-terminal or central region, like inside the amino acid sequence 1-28 of the beta amyloid protein, e.g. it could recognise epitopes corresponding to the amino acids or comprised between amino acids 1-5, 1-16,17-24,13-28. Also the C-terminal region could be subject to recognition, for instance the sequence comprised between amino acids 33-42 or 33-40. Examples of commercially available antibodies that could be used for the elaboration of the calibration curve are 4G8, 6 E10, G2-11, 3D6, m266, Solanezumab, Ponezumab, Bapineuzumab, Gantenerumab, BAN2401, BIIB037, intravenously administered immunoglobulins (IVIG) such as Gammagard, Octagam, Newgam.

For the realization of the present invention, the antibody having a known titre could be an antibody belonging to any species, for instance a murine antibody belonging to the IgG2a or IgG2b class, a human antibody or a humanized antibody belonging to the IgG class. In an embodiment, this human or humanized antibody could belong to one of the classes IgG2a, IgG2b, or IgG1. The use of antibodies belonging to these classes for the elaboration of the calibration curve will prove particularly interesting when utilizing protein A or protein G as secondary antibody, since proteins A/G have a greater affinity for their Fc region.

The calibration curve elaborated with the antibody of known concentration could then be used to determine antibodies concentration antibodies in samples with an unknown titer. For instance, the reading of the absorbance or fluorescence signal obtained with the samples having a known titre at different concentrations will enable to elaborate a regression curve from which to extrapolate the concentration of antibodies in the sample of CSF of unknown titre that is being analysed according to conventional methods well-known to the technician in the field.

In an embodiment, the invention could therefore provide an *in vitro* method for monitoring the response to the active or passive immunisation therapy in a patient suffering from Alzheimer disease.

One of the strategies for the treatment of Alzheimer disease is the active and/or passive immunisation of the patients suffering from this disease. Active immunisation envisages administration of Abeta peptides (or analogues thereof) to the patients, whereas passive immunisation envisages administration of humanized antibodies against the beta amyloid protein. To be able to monitor the therapeutic effect obtained with this type of therapies it might be necessary to dose with extreme precision and accuracy the quantity of autoantibodies in the CSF before, after and during the treatment.

In clinical research, autoantibodies dosage in the CSF might prove extremely useful for the selection of subjects suitable as candidates for clinical trials, enabling a better stratification at admission and a better monitoring of the patient during the trial itself. The possibility of dosing such antibodies preliminarily and/or during the applying of a therapeutic method makes it possible not only to monitor the pharmacological effect thereof at central level (in the CSF), but also to derive from such monitoring useful information for the improving of the therapeutic strategy, enabling better treatment customization and safety. Such a customization could be carried out, e.g., by modifying the therapeutic dosages of the therapeutic immunisation treatment depending on the progression of antibodies concentration in the CSF (a mechanism to date deemed accountable for the therapeutic effect, but also for the possible appearance of adverse effects such as ARIA).

Detection and dosage of antibodies anti-beta amyloid protein in the CSF are certainly a first fundamental step for monitoring the possible effectiveness of treatments against Alzheimer disease and increasing their therapeutic safety.

Therefore, the present invention provides an *in vitro* method for the monitoring of an active and/or passive immunisation treatment of the Alzheimer disease, and of all Abeta-DMT in general, such as, e.g., inhibitors of Secretase accountable for Abeta production, or of therapies addressed to the tau protein, from samples of CSF from a patient, comprising the above-described steps.

The *in vitro* method for the monitoring of an active and/or passive immunisation treatment of the Alzheimer disease according to the invention therefore comprises the carrying out of the steps of the quantitative method (according to any one of its embodiments) of the invention as described herein, carried out e.g. on plural CSF samples collected in different moments of said treatment. For instance, samples could be collected before, during (n times) and/or after the immunisation treatment, and quantification data obtained by the quantitative method of the invention for each sample could be compared with each other, thus constructing a progression in time of the concentration of antibodies against the beta amyloid protein in the CSF.

The quantitative method of the present invention could also be utilized as a method for the diagnosis of cerebral amyloid angiopathy (both CAA and CAA-ri) and also as a valid tool for the monitoring of the therapeutic progression in these pathologies, such as to evaluate the effectiveness of a steroid treatment thereof.

Cerebral amyloid angiopathy-related inflammation (CAA-ri) is a rare pathology sharing many aspects with Alzheimer disease, in particular in terms of cerebro-vascular Abeta deposition. It has recently been demonstrated (*Di Francesco et al. 2011*) that in patients suffering from CAA-ri an autoimmune phenomenon against Abeta may be detected, with a possible acute and sometimes lethal rise of the levels of autoantibodies against Abeta; the method of the present invention is therefore extremely useful to diagnose the pathology, in association with RMN, in these subjects, thereby avoiding a highly invasive intervention such as a cerebral biopsy.

A further object of the present invention is an *in vitro* method for the diagnosis or therapeutic monitoring of cerebral amyloid angiopathy-related inflammation (CAA-ri) from a CSF sample of a patient comprising the steps of any one of the above-described embodiments.

The method of diagnosis could provide a step in which a determined concentration value is compared to a standard reference value (e.g. deriving from a significant set of healthy individuals) and, when above this threshold value, indicates that the disease is present.

The standard reference value or threshold (cut-off) value of antibodies anti-Aβ could be determined, e.g., by ROC (Receiver Operating Characteristic) curves obtained by elaborating the concentrations of a (statistically significant) set of samples from healthy individuals and individuals suffering from CAA-ri. By said elaboration, for instance threshold values ranging between 30 and 40 ng/mL, like e.g. of 32, 33, 34,22, 35 ng/mL, were determined.

Moreover, clinical trials for the treatment of the Alzheimer disease with antibodies anti-beta amyloid protein (like, e.g., the humanized antibody Bapineuzumab) highlighted that some of the subjects enrolled in the clinical trial had developed vasogenic oedema (VE) after administration of the therapeutic agent. VE therefore appears to be a very serious side effect, occurred more commonly in subjects treated with high doses of the antibody and who are APOE-4 allelic carriers, which led to therapeutic protocols suspension and re-checking. One of the hypotheses for explaining the pathogenetic link between antibodies anti-Abeta administration and VE seems to be an anomalous immune response directed toward cerebro-vascular Abeta deposition, in particular in patients with a predisposition to cerebral-vascular damage, according to a mechanism that might be analogous to what described also for CAA-ri.

Therefore, the present invention provides a method for monitoring an active and/or passive immunisation treatment of the Alzheimer disease comprising the steps of measuring and quantitative comparison of the antibody anti-beta amyloid in the CSF as described above, and wherein also the TFPI in the CSF is measured.

Analogously to the above, the present invention provides a method of aid to the diagnosis and for the monitoring of the treatment of CAA and CAA-ri, comprising the steps of measuring and quantitative comparison of the antibody anti-beta amyloid in CSF as described above, and analogous steps in which also the TFPI in the CSF is measured. TFPI protein, in fact, is an endothelial damage marker whose increase over time in the CSF of monitored patients might indicate an increase of VE risk during the therapy against Alzheimer. The presence of such an increase, therefore, may be a warning light for the extremely dangerous side effects described above.

The method of diagnosis of CAA and CAA-ri could provide also a step wherein the determined concentration value of TFPI is compared to a standard reference value and, when above this threshold value, indicates that the disease is present. Also the threshold (cut-off) value of TFPI could be determined, e.g., by elaborating the ROC (Receiver Operating Characteristic) curves obtained with the concentrations of a (statistically significant) set of CSF samples from healthy individuals and individuals suffering from CAA-ri. By said elaboration, for instance threshold values ranging between 0.70 and 0.90 ng/mL, e.g. of 0.75, 0.80, 0.82 , 0.85 ng/mL, were determined.

The quantitative method of the present invention could also be utilized as a method for the diagnosis or the monitoring of the appearance of radiological abnormalities (ARIA). As shown in Figure 15 and in the experimental section, it has now been discovered that the concentration of autoantibodies against the beta amyloid protein in the CSF of patients is linked to the development of radiological abnormalities related to AD and to CAA-ri, commonly referred to as ARIA. The method of diagnosis could provide a step wherein the determined concentration value is compared to a standard reference value (e.g., deriving from a significant set of healthy individuals) and, when above this threshold value, indicates that radiological abnormalities (ARIA) will develop. In particular, the method could be used in high-risk subjects, like those with greater Abeta deposition, or ApoE epsilon4 genotype carriers, or again in subjects with other risk factors, such as an increased concentration of bio-endothelial damage marker TFPI. In an embodiment of the method of diagnosis of CAA-ri and of ARIA in other pathologies like Alzheimer, concentration of both antibodies anti-Aβ and TFPI will be determined and compared to the threshold values as described above. In this embodiment, by monitoring both parameters a 100% sensitivity and diagnostic specificity could be had, as demonstrated by diagnostic predictivity by ROC analysis and determination of cut-offs obtained.

TFPI dosage in the CSF could be carried out by immunoenzyme assay, like e.g. an ELISA sandwich comprising the following steps:
*I) providing a solid phase coated with an antibody specific for human TFPI;*
   Said solid phase could be a solid phase conventionally used in immunoenzyme assays or immunoassays, coated with antibodies recognising the whole sequence, alternative sequences, truncated sequences or parts of human TFPI.
   After immobilisation, to avoid background noises the solid phase is properly blocked with methods them also conventional, like by treatment with a phosphate buffer solution (pH 7.4) with 1% bovine albumin, 5% serum and 0.05% Tween.
   The incubation time of the blocking solution will be easily identifiable by the average technician in the field and could, e.g., be of about 30, 40, 50, 60, 90 minutes. To remove the unbound material, the solid phase could be rinsed once or more with suitable conventional washing buffers, like PBS + 0.05% Tween.
*II) incubating said solid phase with the CSF sample to be analysed;*
   The incubation time will be easily identifiable by the average technician in the field and could, e.g., be of about 30, 40, 50, 60, 90 minutes. To remove the unbound material, the solid phase could be rinsed once or more with suitable conventional washing buffers, like PBS + 0.05% Tween.
*III) incubating the solid phase obtained in step II) with a protein conjugated to an enzyme, or a radioactive label capable of binding human antibodies, followed by rinsing to remove said unbound conjugated protein;*
   In case an ELISA assay has been carried out, according to the present invention, the protein could be, e.g., conjugated to an enzyme such as horseradish peroxidase (**HRP**) or alkaline phosphatase (**ALP**) or beta galactosidase.
*IV) detecting the signal of said conjugated protein.*

In case of an immunoenzyme assay, after washing to remove the excess of unbound conjugated protein, a solution of suitable substrate, selected according to conventional procedures based on the enzyme conjugated to the protein, will be added in the well in order to enable development of the signal which could then be quantified according to standard techniques. The signal could for instance be quantified by absorbance or fluorescence reading with a suitable tool, depending on the type of enzyme and related substrate used. Signal strength is proportional to the quantity of antibodies present in the sample to be analysed.

In case of a radioimmunoassay, after centrifugation and supernatant settling, or after the step of washing in case of RIA or IRMA assay with antibodies adhering to the solid phase, precipitate radioactivity is counted through a gamma counter. Radioactivity strength will be proportional to the concentration of the specific antibody present in the sample deriving from the patient. Antibody concentration could then be quantitatively measured through a calibration curve.

In an embodiment, the method of the present invention will provide the simultaneous quantification of antibodies against protein TFPI and antibodies anti-beta amyloid protein present in the CSF sample (CO-ELISA assay).

In this embodiment, the solid phase will be coated with both the beta amyloid protein and the antibodies directed against the TFPI protein, and the conjugated protein utilized in step iii) should possess a different wavelength to enable a co-reading of the Ig binding the beta amyloid protein and of the TFPI.

Experiments and examples aimed at better illustrating what has been reported in the present description are reported hereinbelow; such examples in no way should be construed as limitative of the present description and of the subsequent claims.

The present invention also relates to a method of therapy and/or of monitoring for Alzheimer disease, providing one or more steps of quantification of antibodies anti-beta amyloid protein in samples of cerebrospinal fluid from the patient or patients to be treated and/or in treatment according to the present description, said method optionally comprising also one or more steps wherein for each of said samples the concentration of one or more endothelial damage marker, like, e.g., of TFPI, is determined according to standard methods applied to the CSF as described herein.

All of what has been described above in connection with the method of quantification of antibodies anti-beta amyloid protein in samples of cerebrospinal fluid and of one or more endothelial damage markers (like e.g. of TFPI) applies to the method of therapy and/or of (prognostic) monitoring of the invention.

### EXAMPLES

### EXAMPLE 1 Procedure for concentrating with magnetic micro beads

1) Wash twice with PBS, pH 7.4, + 0.02% Tween, 75 µl of magnetic micro beads conjugated to protein A (Invitrogen Dynal AS Dynabeads® protein G) for each sample to be analysed;
2) add to the washed magnetic micro beads a sample volume comprised between 200 and 500µl of CSF from a patient and incubate under stirring for 40 min. at room temperature;
3) place the magnetic micro beads in the magnet for 2 min.; optionally store supernatant in another test tube (it will act as control for process efficiency);
4) remove from magnet and wash with 500 µl of citrate-phosphate buffer, pH 5 + 0.02% Tween, repeat twice;
5) then proceed with the elution phase, by adding 30 µl of 0.1M citrate at pH 3.1 to the magnetic micro beads;
6) stir for 2 min;
7) place the tube in the magnet for 1 min and immediately transfer supernatant in a clean test tube (Eppendorf polypropylene low affinity tube) containing 40 µl of 1M Tris Base, pH 9 (buffer solution);
8) repeat steps 5), 6) and add eluate to the same test tube used at 7) (total volume: 100 µl);
9) use all 100 µl of eluate for the ELISA.

### EXAMPLE 2 Coating the microplate with Abeta42 protein

1) dissolve Abeta42 protein in 1M Tris Base buffer, pH 9, to a concentration of 1 µg/µl;
2) prepare a coating solution of Abeta in 50 mM NaHCO₃, pH 9.6, to a concentration of 0.01 µg/µl;
3) sonicate for 1 min on ice, wait 1 min, sonicate again for 1 min;
4) add 100 µl of a coating solution per ELISA plate well (end concentration: 1 µg/µl);
5) incubate overnight.

### EXAMPLE 3 immunoenzymatic dosage

1) on the day following the coating performed as described in example 2, carry out 3 washings with PBS + 0.05% Tween (PBST), 200 µl/well;
2) perform blocking to exclude aspecific binding, by using a solution comprised of 5% serum and 1% BSA in PBST, 1.5 h at room temperature under oscillation;
3) repeat the washing step described at 1);
4) add 100 µl of the solution containing the primary antibody 4G8 (directed toward the amino acid portion 17-24 of the Abeta protein), to construct the standard curve. Standards are carried out by serial dilutions of the primary antibody. The range suggested for the standard curve for antibodies dosage in the CSF is of from 0.24 to 0.03 µg/ml. In wells to be used as blank, add only 100 µl of PBS. Moreover, a further blank for eluates is provided, comprised of 60 µl of 0.1 M citrate, pH 3.1, plus 40 µl of 1M Tris Base, pH 9.
5) add 100 µl of the sample of CSF as concentrated in Example 1 into the wells. Leave under stirring at 4°C overnight.
6) repeat the washing step described at 1);
7) add 100 µl of the solution containing protein A-HRP at a concentration of 0.2 µg/mL Leave under stirring for 2 h at room temperature.
8) repeat the washing step described at 1);
9) add 100 µl per TMB well. Incubate in the dark for a period of about 5-10 min, at room temperature.
10) add 100 µl per well of a solution having an acidic pH, such as 0.5N HCl
11) read absorbance of each well at 450 nm, then subtracting the value of the blank (well in which neither the standard, nor the unknown sample were added). After extrapolation of the unknown sample from the standard curve, adjust value depending on the initial dilution and/or concentration factor.

### Example 4 Preparing ELISA standards and reagents

The antigen, comprised of Abeta protein (Abeta 40 or Abeta42 or their respective different oligomeric forms) are adsorbed (coating) on the bottom of wells of an ELISA microplate. During the first incubation, the standards comprised of known concentrations of antibodies anti-Abeta of murine origin, controls and unknown CSF samples to be analysed are distributed in the wells with Abeta and incubated overnight.

Subsequently, after washing with PBS buffer, protein A conjugated with HRP (prot A-HRP), acting as detector for antibodies that have specifically bound during the first incubation, is added.

After washing to remove the excess of unbound protein A-HRP, a substrate solution is added into the well, in order to enable development of stain which will subsequently be quantified by absorbance reading, or possibly by fluorescence reading. In fact, the strength of this stained product is directly proportional to the concentration of antibodies anti-Abeta present in the original sample, extrapolated from a standard calibration curve at known concentrations.

### Example 5 Comparative tests

We tested the different sensitivity for plasma dosage of published ELISA, which used clone G2-11 as primary antibody, or clone 3D6 in the publication of *Dodel et al 2001* or *Conti et al 2010*, compared to an embodiment of the method of the present invention.

Specifically, antibodies of clones G2-11, 6E10 and 4G8 were tested as primary antibodies, in order to identify which one might be the best to associate to our ELISA method.

By testing a solution having a known concentration (0.25 ug/mL) of antibody anti-Abeta 4G8, our new ELISA proved 3.5 times more sensitive, in terms of absorbance concerning the highest points of the curve, compared to the use, e.g., of the previously published clone G2-11 (1.469 vs 0.426) (see Figure 3).

Clone 4G8 yielded the best results, proving to be the best above all when associated to the novel detection system (protein A-HRP).

By way of example, in Figure 3 a table is reported, indicating the results, in terms of Absorbance (OD), obtained by dosing the same sample having a known concentration of antibodies with the old ELISA (i.e., by using the detection system with an anti-goat IgG (Anti-Human IgG (whole molecule)-Peroxidase antibody SIGMA #A8667) left-side column, compared to an embodiment of the method of the present invention (by using Protein A conjugated to HRP, right-side column).

Figures 4A and 4B show the different sensitivity of the standard curve in the detection of samples having low concentrations of antibodies. In fact, the dosage of 3 different samples of CSF deriving from an AD patient, a CAA-ri patient and a control, after a suitable treatment of concentrating with micro beads as described by the Inventors, proved totally ineffective by using the method described in the state of the art, whereas the novel ELISA method (4G8 + protA-HRP) of the Inventors proved highly sensitive and effective. In fact, all three samples analysed fell perfectly within the linearity range of the standard calibration curve, as evidence of the effectiveness of our novel technique as "ultra-sensitive" method for the dosage of antibodies anti-Abeta in the CSF.

Moreover, Figure 4C shows how for a typical calibration curve the novel ELISA method described herein be actually 10 times more sensitive compared to the old ELISA method in the detection range expected for the dosage of the antibodies anti-Abeta in the CSF (range of between 0.14 and 0.04ug/mL).

In fact, the present invention exhibits a calibration curve capable of detecting antibodies concentration in a curve linearity range of between 0.18 and 0.03 ug/mL, whereas the old ELISA method described in the state of the art with antibody G2-11 proved capable of quantifying samples with a much higher concentration, therefore being ineffective in the CSF.

It may be inferred how, in terms of absorbance, at the comparable concentration of 0.134 ug/mL, the OD value detectable with our novel ELISA method is 1.072, compared to the 0.142 detectable with the ELISA method described in the state of the art, i.e. about 10 times more sensitive, whereas for all standards lower than 0.134 ug/mL the preceding method was not capable of discriminating any value from the blank.

Moreover, we tested and ruled out possible interferences given by the matrix effect. In fact, untreated CSF and CSF after concentration with micro beads returned absorbance values identical to those of an ELISA well in which Abeta protein had not been adsorbed, and anyhow comparable to the blank.

Likewise, lack of interference of HRP-conjugated protein A with the Abeta coating was tested for and confirmed. The pre-enrichment stage moreover proved useful also in terms of background reduction, returning decidedly lower values as "blank" (Absorbance of 0.077, compared to of 1.11 with the old ELISA). Then, the minimum volume indispensable for dosing anti-Abeta 42 autoantibodies in the CSF of control subjects, like normal pressure hydrocephalus, in which antibody concentration should be physiologically among the lowest expectable ones, was tested. The volume of CSF utilized for the concentration stage in the controls was of between 400-500 µl.

Said initial CSF volume of 500 µl was then treated with 75 µl of magnetic micro beads conjugated with protein A, and eluted (stage of CSF Ig specific enrichment) with 60 µl of 0.1M citrate solution, pH 3.2, and immediately additioned of 40uL of buffer solution (1M TRIS Base, pH 9) in order to preserve Ig native conditions. The entire resulting volume (100 µl) was then utilized for the ultra-sensitive ELISA dosage invented by us.

Therefore, the mean concentration of autoantibodies against Abeta protein in the control subject, adjusted by the concentration factor, proved to be of about 16 ng/ml.

### Example 6 Precision parameters and recovery test of the method according to an embodiment:

Intra-assay precision parameters, i.e. the variability inside the same microplate of returning comparable values for repeated readings, were assayed by exact reproduction of the procedures described in the preceding Examples 1, 2 and 3.

Intra-assay precision (mean obtained by dosage in triplicate of 3 different samples of CSF coming from 3 subjects) = CV 3.27%

Inter-assay precision parameters, i.e. the method variability of returning comparable values for repeated readings during plural assays performed in different days, were assayed by exact reproduction of the procedures described in the preceding Examples 1, 2 and 3.

Inter-assay precision (mean obtained by dosage, repeated 7 times in 5 different ELISA, of the same sample of CSF coming from the same patient) = CV 3.69%

Moreover, intra-assay precision of the sole step of concentrating with the magnetic micro beads was tested (i.e. by exact reproduction of the procedures described in example 1) with 3 different enrichments of the same sample performed in consecutive days, frozen at -20° C, and then dosed in the same ELISA following the exact reproduction of the procedures described in Example 2) and 3) = CV 2.33%

The recovery test (performed by addition (spiking) of 0.125 µg/ml of antibody 4G8 in 500 µl of CSF subjected to all enrichment steps as described in examples 1), 2) and 3)) proved to be 98%.

As negative control, the supernatant (discard) obtained as described at 3) of Example 1) "procedure for concentrating with magnetic micro beads" was tested. The procedure proved 100% effective for all tests performed (> 20 repeated tests) returning each time a concentration value equal to 0, i.e. to the blank.

Finally, also test specificity was tested, by coating some wells of the ELISA plate with the same concentrations utilized for the standard calibration curve (see above), in which however Abeta42 was replaced by scrambled Abeta42 (Anaspect), i.e. a synthetic peptide comprised exactly of the same amino acids making up the Abeta42 protein, but in open order, in a 1M Tris Base buffer, pH 9. The abovementioned test demonstrated that in said wells no signal was detectable, as evidence of a 100% specificity of our method toward Abeta40 and Abeta42.

### Example 7 Measuring by immunoenzyme assay of TFPI in CSF samples from AD patients

In this specific example, an immunoenzyme assay ELISA comprising 96-well plates coated with a murine antibody specific for TFPI (Quantikine Human TFPI ELISA - R&D Systems) was used. Different ELISA may be constructed in-house, e.g. specific for the different truncated forms or isoforms of TFPI. Specifically, the present KIT is not marketed for TFPI dosage; therefore, after having approved its specificity and sensitivity (results not shown) we proceeded as follows, by modifying the protocol indicated by the manufacturing company:
1) The provided standard was diluted with suitable diluent provided for constructing the standard curve (according to the manufacturer's indications) which thereafter would serve for extrapolating the unknown value of the sample;
2) 100µl of diluent buffer provided in the kit were added into each well;
3) 50µl of Standard or of sample (CSF) diluted 1:2 were immediately added;
4) Incubated at room temperature for 2h;
5) 4 washings were performed with 300µl of Wash Buffer, inverting the plate on blotting paper to completely eliminate the liquid each time;
6) 200µl of secondary antibody (Conjugate, provided in the kit) were added, incubating for 120 minutes at room temperature;
7) 4 washings were performed with 300 µl of washing buffer, inverting the plate on blotting paper to completely eliminate the liquid each time;
8) 200 µl of substrate solution (substrate solution provided in the kit) were added, incubating for 30 minutes at room temperature, in the dark;
9) 50 µl of stop solution were added into each well. Colour would turn from blue to yellow. Immediately, absorbance at 450nm was read. Sample absorbance was then multiplied by 2, factor by which it had been diluted.

### 8 Study related to anti-Aβ autoantibodies involvement in CAA-ri development.

### 8.1 Materials and methods - Patient enrolment

Samples of CSF coming from 10 patients (7 Italians, 2 cases from Brazil, 1 from Japan) suffering from CAA-ri were recruited through an International collaboration. Probable CAA-ri diagnosis was performed on the basis of clinical and neuroimaging features known in the literature, but in blind with respect to CSF concentration of anti-Aβ autoantibodies.

All CAA-ri patients exhibited acute or sub acute neurological symptoms comprising mental confusion, convulsions and focal neurological deficits. Cerebral magnetic resonance, evaluated during the acute phase, showed signs of white matter inflammation consistent with vasogenic oedema (FLAIR sequences) and with the presence of micro bleeds more scattered throughout the cerebral cortex (T2*-GRE). Moreover, cases 5 and 6 were also confirmed by stereotactic brain biopsy, showing Aβ deposition around cerebral vessels and neuroinflammation signs. APOE genotype analysis was performed for all subjects, according to standard procedures adopted at each recruiting centre.

CSF samples from the CAA-ri patients were collected during the acute phase of the disease (apCAA-ri) and immediately stored at -20° C. CSF aliquots from 7 patients were collected even after steroid treatment (rpCAA-ri), whereas for cases 6 and 8 a CSF sample was collected even after spontaneous resolution of symptoms, therefore in the absence of any pharmacological treatment.

As control subjects for the presence of cerebral amyloid angiopathy, but without neuroinflammation signs, we collected CSF from 8 patients suffering from non-inflammatory CAA (diagnosed according to the Boston criteria, Knudsen 2001). As control group of autoimmune and inflammatory, but CAA-correlated disease, we included 14 naive patients suffering from multiple sclerosis (MS), with positive oligoclonal bands (OCB+), diagnosed according to McDonald's criteria. As non-CAA, non-inflammatory controls, we included 25 CSF coming from 12 normal pressure hydrocephalus subjects and 13 patients with conversion disorder.

### CSF collection and AB40, AB42, tau and P-181 tau dosage modes

All CSF were collected by lumbar punture at L4/L5 or L3/L4 vertebral level, divided into 0.5 mL aliquots each in polypropylene tubes and immediately stored at - 20°C until analysis time. Count of cells present in the CSF, glucose and total proteins were evaluated. Aβ40, Aβ42, tau and P-181 tau concentrations were evaluated by commercial ELISA kits (respectively Millipore and Innogenetics), in accordance with manufacturer's instructions.

### Anti-Aβ autoantibodies dosage

For autoantibodies dosage, the *in vitro* method for the quantification of antibodies anti-beta amyloid protein in a sample of cerebrospinal fluid described herein was used. By said method, the concentration of antibodies naturally produced against Aβ was evaluated by pre-enrichment with magnetic beads and subsequent antibody dosage with ELISA method. To increase the sensitivity of the ELISA technique, samples were concentrated by pre-treatment with magnetic beads, in order to enrich total IgG content. Briefly, about 200-300 µl of CSF were incubated with magnetic beads conjugated with protein G (Dynebeads Protein G) for 40 min, under continuous stirring at room temperature (r.t.), and eluted by slight acidification with 0.1 M citrate (pH 3.1). After magnetic enrichment, eluates were directly tested by our novel ELISA method. The wells of a 96-well plate were coated with Aβ42 (Phoenix Pharmaceuticals, Belmont CA, USA), 1 pg/well, dissolved in 50 mM carbonate buffer (pH 9.6) at 4 °C, overnight. The plate was then washed with PBS/0.05% Tween 20 (PBST), blocked with 5% FCS, 1% BSA in PBST for 90 min, at room temperature, and washed again. For the generation of the standard curve, an antibody anti-human Aβ42, produced in mouse and selective for the N-terminal of Abeta (clone 4G8, Covance), was utilized at different concentrations, by serial dilutions (0.4 ug / ml to 0.03 ug / mL). After loading the samples and standards, the ELISA plate was then incubated overnight, at 4°C. On the following day, wells were washed with PBST and incubated for 2 hours with HRP-conjugated protein A (Millipore), at r.t. Then, the plate was washed again with TBST and incubated for 10 min with TMB, in the dark. After addition of the stop solution, absorbance at 450nm was read by using a microplate reader (BIORAD Model 550). Autoantibodies concentration was extrapolated from the standard curve.

All samples were analysed in blind, without the operator knowing the diagnosis. As quality control of the process of enrichment with magnetic beads, all supernatants (process discards) were tested in order to confirm total depletion of IgGs by the beads. ELISA specificity toward human Aβ was verified by exclusion of cross-reactivity toward Scrambled Aβ1-42 peptide (ANASPEC, Fremont, CA, USA), by coating of 1ug of peptide. Anti-Aβ antibodies recovery in the CSF matrix was evaluated by spiking test of different concentrations of the commercial antibody 4G8 at the different concentrations used to generate the standard curve of the ELISA, obtaining a 98% recovery. Intra-assay precision proved to be 3.3% of the Coefficient of variation (CV); inter-assay precision proved to be 3.7% of the CV.

### Statistical analysis

Statistical analysis was carried out with GraphPad-Prism software (Prism 3.0, GraphPad Software, Inc.). To evaluate inter-group differences, one-way analysis of variance test was used, followed by Tukey's multiple comparison test, or an unpaired t test was used for the analysis of data groups that passed a normality test. A paired t test was used to evaluate differences in antibody concentration before and after clinical remission. Values with a p < 0.05 were considered significant. Continuous variables are expressed as mean ± standard deviation (MEAN±SD).

### 8.2 Results, patients' features and histopathological analysis

Age proved comparable between the different groups of subjects: healthy controls (59.0 years ± 20.0), CAA (59.6 years ± 11.4), MS (52.5 years ± 12.2), CAA-ri (68.5 years ± 5.6). Male/Female number proved to be: healthy controls 12/13, CAA 5/3, MS 11/3, CAA-ri 3/7.

A socio-demographic analysis of CAA-ri patients is reported in Table 1, whereas clinical features and adopted pharmacological treatment of every CAA-ri patient are reported hereinafter:
Case 1: Progressive behavioural disorder and dementia. Treated with methylprednisolone 1 g /day for 5 days, with a slight benefit.
Case 2: progressive cognitive decline and generalized attacks. Treated with methylprednisolone, 1 g/day for 5 days and subsequently 1 g/week for 7 weeks, with a moderate clinical improvement.
Case 3: progressive loss of memory and mood disorder. Treated with dexamethasone 24 mg/day for 20 days, with a marked clinical improvement, as previously described.
Case 4: rapid and progressive left-sided hemiparesis and disorientation. Treated with dexamethasone 24 mg/day for 1 month, subsequently discontinued due to onset of pneumonia. Deceased thereafter owing to infective complications.
Case 5: progressive left-sided hemisyndrome with partial homolateral attacks. Initially treated 6 times with methylprednisolone, 1 g/day for 3 days, followed by oral therapy with methylprednisolone. Marked clinical improvement as previously reported.
Case 6: progressive cognitive decline and mental confusion. Spontaneous remission of symptoms without any pharmacological treatment.
Case 7: Mild transient cognitive decline. Spontaneous remission of symptoms without any pharmacological treatment.
Case 8: Transient mental confusion. Spontaneous remission of symptoms without any pharmacological treatment.
Case 9: Transient mental confusion and generalized attacks. Treated with prednisone 80 mg/day for 10 days, with remission of symptoms.
Case 10: Progressive cognitive decline and parkinsonism. Treated with dexamethasone 12 mg/day for 15 days, then progressively reduced for other 2 weeks, without any significant improvement.

**Table 1. Socio-demographic and clinical features of the CAA-ri patients.**

| **Patient #** | **Gender (M/fF** | **Age (a.o.)** | **APOE** | **Country** | **Treatment** | **rpCAA-ri CSF re-collection** |
|---|---|---|---|---|---|---|
| 1 | F | 68 | ε3/ε4 | Brazil | I.V: steroid | Yes |
| 2 | M | 62 | ε3/ε3 | Brazil | I.V: steroid | Yes |
| 3 | M | 68 | ε4/ε4 | Italy | I.V: steroid | Yes |
| 4 | F | 76 | Not tested | Italy | I.V: steroid | Yes |
| 5 | M | 56 | ε3/3ε | Japan | I.V: steroid | Yes |
| 6 | M | 71 | ε3/3ε | Italy | No treatment | Yes |
| 7 | M | 72 | ε4/ε4 | Italy | No treatment | No |
| 8 | F | 72 | ε3/3ε | Italy | No treatment | Yes |
| 9 | M | 71 | ε4/ε4 | Italy | Oral steroid | No |
| 10 | M | 67 | ε4/ε4 | Italy | I.V: steroid | no |

| | | | | | | |
|---|---|---|---|---|---|---|
| APOE, apolipoprotein E; a.o., age at disease onset; rpCAA-ri, patient with cerebral amyloid angiopathy in remission phase. | | | | | | |

**Table 2 reported below shows in more detail socio-demographic and clinical features of CAA-ri patients.**

| Case # | Age Gender | APOE | Clinical aspects | | | Neuroradiological aspects | | | | | |
|---|---|---|---|---|---|---|---|---|---|---|---|
| | | | Acute phase (LQR collection from onset) | Treatment | Remission (LQR collection from onset) | Acute phase | | | Remission | | |
| | | | | | | ARIA-E (FLAIR) | C.E. | ARIA-H (T2*-GRE) | ARIA-E (FLAIR) | C.E. | ARIA-H (T2*-GRE) |
| **1** | 68/F | ε3/ε4 | mood disorder, cognitive decay, agitation (10 m.) | MPS 1g/d IV 5 d | Reduction of cognitive decay and agitation (11 m.) | Multiple areas, bilateral, mainly Fl | no | > 70 | Little reduced | no | unvaried |
| **2** | 62/M | ε3/ε3 | cognitive decay, visual hallucinations, GTC crises (6 m.) | MPS 1g/ IV 5 d | Reduction of cognitive decay, hallucinations and crises (7 m.) | Multiple areas, bilat, mainly Fl and Pl | no | > 130 | Little reduced | no | unvaried |
| **3** | 68/M | ε4/ε4 | memory loss, mood disorder (4 m.) | DMX 24 mg/d IV 20 d | Remission of memory loss and mood disorder (7 m.) | 3 greater swellings Lt Fl, Tl-Pl, Rt Pl | no | > 200 | Reduced | no | unvaried |
| **4** | 76/F | N/A | left-sided hemiparesis, cognitive decay (2 m.) | DMX 24 mg/d IV 30 d | Near-total remission of hemiparesis and cognitive decay (4 m.) | 2 greater swellings TI-PI bilat | Yes, leptomeningeal | 26 | Very reduced | no | unvaried |
| **5** | 56/M (biopsy) | ε3/ε3 | left-sided hypoesthesia in partial crises (3 m.) | MPS 1 g/d IV 3 d-6 times | Remission of sensitive disorder and crises (7 m.) | 1 greater swelling Rt Pl | Yes, leptomeningeal | no | Very reduced | no | no |
| **6** | 71/M (biopsy) | ε3/ε3 | Cognitive impairment and mental confusion (5 m.) | no | Near-total remission of cognitive impairment and confusion (8 m.) | Multiple, mainly Rt PI and bilat TI | Yes, sulcal leptomeningeal | N/A | Very reduced | No | 50 |
| **7** | 72/M | ε4/ε4 | cognitive decay (2 m.) | No | Near-total remission of cognitive decay (N/A) | Giant Rt Tl-Pl-Ol | Only 1, sulcal | 55 | Very reduced | No | unvaried |
| **8** | 72/F | ε3/ε3 | mild cognitive decay in GTC crises (2 m.) | No | Near-total remission of cognitive decay, crises disappearance (5 m.) | Moderate, bilat Fl,Rt posterior | No | 50 | Little reduced | No | Univaried Unvaried |
| **9** | 71/M | ε4/ε4 | GTC crises and transient cognitive decay (1 m.) | PRD 80 mg/d oral 15 d | Remission of cognitive decay and crises (N/A) | Lt FI,Rt PI-TI-OI | Minimal, leptomeningeal | > 500 | Reduced | no | unvaried |
| **10** | 67/M | ε4/ε4 | disorientation, cognitive decay, scanning speech, expressive face (3 m.) | DMX 32 mg/d IV 15d | Reduction of behaviour symptoms, parkinsonism signs unvaried (N/A) | Multiple areas, bilateral, mainly Rt PI | Marked, leptomeningeal | 10 | Reduced | no | unvaried |

### Histopathological analysis

As reported in Figure 6, CAA-ri diagnosis was immunohistologically confirmed in 2 patients: for case #6, patient not treated with immunosuppressant, Aβ deposition around cerebral vessels and presence of signs of neuroinflammation typical of CAA-ri were confirmed. Case #5 had already confirmed the presence of a pathological picture of CAA-ri in the bioptic analysis, as previously reported in Sakaguchi et al. 2010.

### CSF dosage of anti-Aβ autoantibodies

As shown in Figure 8a, anti-Aβ autoantibodies concentration in the CSF of patients suffering from CAA-ri during the acute phase (52.8 ng/mL ± 21.9) proved significantly increased compared to: healthy controls (18.1 ng/mL ± 5.7; p<0.0001), non-inflammatory CAA controls (24.0 ng/mL ± 6.6; p<0.0025) and MS patients (19.7 ng/mL ± 2.5; p<0.0001). Moreover, the concentration of autoantibodies of CAA-ri patients proved to return to normal values (i.e., comparable to controls) after the remission phase of the pathology (14.5 ng/mL ± 8.8; p<0.0006 vs apCAA-ri). No statistically significant difference emerged between the controls group and CAA or rpCAA-ri (Figure 8a). Moreover, noteworthy is the fact that the same reduction of antibody concentration was observed both after steroid therapy treatment and after a spontaneous remission of the symptoms, i.e. without treatment. In fact, cases 1-5 showed an antibody reduction following (intravenous) steroid treatment, whereas for cases 6 and 8 antibody concentration decrease proved spontaneous. Figure 8b shows the specific trend of pre-/post-remission decrease of antibodies concentration in the CSF, illustrated case wise.

### Time progression of antibodies anti-Aβ during the steroid treatment

Time progression of the change of the concentration of antibodies anti-Aβ in patient #5, in which the diagnosis was confirmed by cerebral biopsy, was monitored. As shown in Figure 9, antibody concentration, compared to the first dosage during the acute phase of disease, exhibited a progressive reduction following each IV administration of steroids, until reaching the concentration levels detectable in control subjects. Antibody reduction follows also the parallel improvement of the clinical picture, as also confirmed my MRI investigation contextual to each CSF collection.

### Aβ40, Aβ42, tau and P-181 tau trends

CSF profile of Aβ40, Aβ42, tau and P-181 tau in apCAA-ri and rpCAA-ri patients was compared. Aβ40 and Aβ42 concentration in the apCAA-ri group proved significantly higher compared to the rpCAA-ri group (2107.0 pg/mL ± 446.4 vs 1202.0 pg/mL ± 618.3, p=0.009; and 640.0 pg/mL ± 338.3 vs 234.5 pg/mL ± 168.8; p=0.015, respectively), reaching the levels detectable in healthy controls (1864.0 pg/mL ± 669.2, p=0.032; and 747.0 pg/mL ± 342.2; p=0.002 vs rpCAA-ri, respectively) and in line with data present in the literature. Tau (335.9 pg/mL ± 312.7) in apCAA-ri patients proved high compared to rpCAA-ri (85.2 pg/mL ± 70.0; p=0.0041) and to healthy controls (131.4 pg/mL ± 115.4; p=0.012), as shown in Figure 5b. P-181 tau (47.0 pg/mL ± 30.5) during the apCAA-ri phase proved high compared to rpCAA-ri (22.8 pg/mL ± 7.6; p=0.007) and controls (29.0 pg/mL ± 16.1; p=0.044). The concentration trend of Aβ40, Aβ42, tau and P-181 tau for each individual patient during apCAA-ri and rpCAA-ri is reported in Figures 10a, b, c and d.

Correlation between concentration of antibodies anti-Aβ and, respectively, CSF levels of Aβ40 and Aβ42.

Correlation analysis in apCAA-ri showed an evident correlation between concentration of antibodies and circulating forms of Aβ40 (r=0.85, p=0.002), and Aβ42 (r=0.65, p=0.042), as depicted in Figure 11.

### Physiological levels of autoantibodies in the CSF of Alzheimer subjects

As shown in Figure 15, the levels of antibodies anti-Aβ present in the CSF of Alzheimer patients were evaluated, observing that patients carriers of the genetic risk factor ApoE epsilon4 (APOE4 carriers) have a greater physiological concentration of autoantibodies (12.84 ng/mL ± 21.23) compared to APOE4 NON-carriers patients (2.874 ng/mL ±3.063; p<0.001), in support of a greater predisposition thereof to develop ARIA, both spontaneously and following Abeta-DMT administration.

### 8.3 Discussion

CAA-ri etiopathogenesis still remains unknown, though the mechanism leading to the inflammatory process seems to be secondary to an autoimmune reaction against protein Aβ present in the CNS. Sporadic observations tend to confirm this hypothesis, with a recruiting of specific CD4+ T cells in the Aβ deposition sites and an increase of antibodies anti-Aβ in the CSF, in spite of these observations having been made in individual clinical cases only, and the dosage of the concentration of antibodies anti-Aβ having been performed with old methods subject to wide margins of error.

Alike speculations have recently been advanced also in AD, although even in this pathology the dosage of auto antibodies anti-Aβ had led to scarce and conflicting results, restricted mainly to plasma level, just owing to technical experimental difficulties in the antibody dosage in the CSF. Actually, the dosage of anti-Aβ autoantibodies in AD patients had proved reduced for some authors, unvaried for others, and even increased in another study. As mentioned, the main problem of this conflicting results was due to the very low concentration of these antibodies in human CSF, besides problems linked to the design and power of the above cited studies.

Thanks to the method described herein for the concentration and dosage of antibodies, we succeeded for the first time to confirm a direct involvement of these antibodies anti-Aβ during the course of CAA-ri, in a case record comprised of 10 patients. Moreover, we demonstrated that antibody concentration actually increased during the acute phase of the illness, compared to healthy control subjects, MS subjects, and to patients suffering from non-inflammatory CAA, until reaching levels that can be considered "normal", i.e. comparable to what is present in the controls, during the phases of clinical and radiological remission.

In addition, by following the time progression of antibodies anti-Aβ during the steroid treatment, we demonstrated a decrease thereof not only following the therapy, but also following a spontaneous remission, as further evidence of a specific role of the rise in the concentration of antibodies anti-Aβ in the determining of the acute phase of disease. This is particularly relevant, as we could definitively prove that this event is not secondary to an aspecific effect of the pharmacological treatment, but strictly correlated to disease progression. This evidence is further supported by the non-presence of an increase of antibodies anti-Aβ in patients suffering from a non-CAA-correlated autoimmune and inflammatory disease, represented by OCB+ MS.

Overall, the data disclosed here strongly support the idea that CAA-ri pathogenesis is caused by a specific autoimmune process toward Aβ protein, directly mediated by anti-Aβ autoantibodies.

The same pathogenetic mechanism might moreover prove fundamental in the determining of the reversible vasogenic edema that can be found in about 5-10% of AD patients enrolled in the clinical trials with the monoclonal antibody directed toward Aβ, termed bapineuzumab, adverse effects that even entailed discontinuance of therapeutic protocols under way. Accordingly, recent evidences obtained by MRI studies with the sequences termed ARIA-E (amyloid-related radiological (imaging) abnormalities suggestive of vasogenic oedema), highlighted that up to 17% of patients treated with bapineuzumab show signs of VE, even in the absence of clinical correlates and in direct connection with the therapeutic dosage and the presence of the vascular risk factor APOE ε4.

In support, Schroeter et al. moreover demonstrated that passive immunisation in PDAPP mice is strictly linked with the incidence of microbleeds, limited to focal Aβ deposition at the perivascular level. It appears clear how this phenomenon could be closely related to the degree of CAA, and modulated (and modulable) by the concentration of the dose of antibodies anti-Aβ. This might mean that a suitable modulation of the concentration of antibodies anti-Aβ administered, capable of maintaining the concentrations thereof within values that can be considered as "safe", might eliminate or at least drastically delay Aβ deposition at cerebral and cerebro-vascular level, without incurring the listed adverse effects, such as VE (ARIA) and endothelial damage.

Moreover, a pathogenetic effect shared by AD and CAA was highlighted by the demonstrated clearance of Aβ protein from the CNS of AD patients during clinical trials with bapineuzumab, suggesting a massive drainage of Aβ from the parenchyma-deposited and/or vascular form to the circulating form, particularly during the first phases of the treatment and before a complete disappearance of the amyloid plaques. This is perfectly in line with what we observed in patients suffering from CAA-ri, where the circulating levels of Aβ40 and Aβ42 proved comparable to those detectable in healthy controls and to apCAA-ri subjects, whereas said levels return to significantly reduced in the remission phases of the disease, until reaching even the concentrations normally detectable in AD patients. Likewise, markers for axonal degeneration (tau and P-181 tau) proved augmented in CAA-ri, together with the dramatic increase of the anti-Aβ autoantibodies, then followed by a returning within the range of healthy controls in rpCAA-ri. This definitively proves that anti-Aβ antibodies are not merely the main actors during the acute phase of CAA-ri, but that the increase of the levels of Aβ40 and Aβ42 in the CSF seems to be the mechanism through which such antibodies act, removing Aβ from the plaques and/or the vessels, a mechanism however further supported by the strong correlations detectable between the levels of amyloid protein and of the antibodies themselves. Moreover, the high prevalence of patients carriers of haplotype APOE ε4 further reinforces the greater susceptibility of these patients toward predisposition to endothelial damage, as previously described also for AD. Considering the high expectations placed in the clinical trials under way for passive immunisation toward Aβ, a better understanding of the pathogenetic mechanisms shared by CAA and AD is fundamental in order to ensure all beneficial effects of the clinical trials in the safest and most effective way possible. In this context, CAA-ri might represent a spontaneous human model of the pathophysiological mechanisms detectable in the AD subjects who exhibited VE during the treatment with bapineuzumab.

Our data represent a significant step forward for an understanding of the pathogenetic mechanisms at the basis of the inflammatory reaction which manifests itself during CAA-ri, opening up, moreover, interesting perspectives for the treatment of all those pathologies correlated to an anomalous processing of the amyloid precursor protein and to Aβ deposition in the CNS, such as CAA and AD. The results foreseeable from our novel method for the dosage of anti-Aβ antibodies in the CSF might then lead to a validation of future and accessible models for the study of pathogenetic mechanisms at the basis of these pathologies and for monitoring the effectiveness of the new amyloid-modifying therapies.

Moreover, starting from our considerations it is possible to surmise future studies correlating the dosage of anti-amyloid antibodies (according to the technique invented by us) with the related MRI data (with the appropriate sequences for ARIA identification) and of deposition of beta amyloid protein with PET, like e.g. with the PET data to C11 Pittsburgh compound B in patients' brains, thereby obtaining directly *in vivo* significant considerations on the removal and deposition of cerebral beta amyloid protein.

Finally, since a final diagnosis of CAA-ri still requires the use of a highly invasive and risky method such as cerebral biopsy, we hope that the use of our method for the dosage of anti-Aβ autoantibodies concentrations in the CSF, in association with the clinical-radiological evidences, may soon be proposed as a valid and alternative biomarker in support of the diagnosis.

### 9 Dosage of TFPI levels in the CSF as index for endothelial damage

The investigation on TFPI levels in the CSF of patients suffering from Alzheimer disease (AD, 0.84 ng/mL ± 0.19) and cerebral amyloid angiopathy-related inflammation (CAA-ri, 1.10 ng/mL ± 0.38) was extended in comparison to levels detectable in a group of healthy control subjects (CTL, 0.48 ng/mL ± 0.29), of controls suffering from a neurodegenerative disease without vascular complications, such as patients with Amyotrophic Lateral Sclerosis (OND, 0.30 ng/mL ± 0.15), and of patients suffering from cerebral amyloid angiopathy without inflammatory component (CAA, 1.04 ng/mL ± 0.57).

As it may be inferred from Figure 12, TFPI concentration in AD, CAA and CAA-ri patients proved significantly increased with respect both to the CTL and the OND (p<0.001, respectively), as evidence not only of the effectiveness of our biomarker at identifying the presence of endothelial damage, but also of a possible direct correlation thereof with amyloid cerebral-vascular deposition levels.

In fact, all 3 categories (AD, CAA and CAA-ri) of patients analysed are pathologically characterised by a strong beta amyloid deposition, both at the parenchymal and cerebral-vascular level.

Moreover, TFPI also proved to be directly correlated to the markers for neurodegeneration and axonal damage, tau and P-181 tau, in patients AD, as evidence that TFPI can represent an index for neuronal death (p<0.0051, r = 0.47; p<0.0041, r = 0.49, respectively) secondary to the degree of amyloid deposition (CAA) (Figure 13).

The clinical and socio-demographic data of the CTL and AD are reported in Table 2, whereas the respective values of CAA and CAA-ri patients were previously reported in Table 1 and 2.

**Table 3. Socio-demographic features of AD patients and related controls utilized for dosage of the TFPI in the CSF.**

| **Variable** | **Controls n=30** | **AD n=44** |
|---|---|---|
| **age (years)** | 70.7 ± 12.0 | 71.9 ± 7.3 |
| **Gender (F/M)** | 17/13 | 22/22 |
| **Adjusted MMSE** | 28.6 ± 1.9 | 21.7 ± 6 # |
| **ApoE4 (% carriers)** | 23.0 | 50.0* |
| **TFPI (ng/mL)** | 0.48 ± 0.29 | 0.84 ± 0.19* |
| **tau (pg/ mL)** | 131.6 ± 104.7 | 743.8 ± 457.6* |
| **P-181 tau (pg/ mL)** | 24.92 ± 14.76 | 65.28 ± 30.34* |
| **Abeta42** | 866.1 ± 209.1 | 428.4 ± 170.5* |
| **Link index** | 0.51 ± 0.07 | 0.56 ± 0.2 |
| **Index of BBB damage** | 7.0 ± 4.7 | 8.0 ± 6.5 |

| | | |
|---|---|---|
| * p<0.0001 vs CTL; # p<0.05 vs CTL. MMSE, Mini Mental State Examination Test. BBB, blood-brain barrier | | |

### 10. Dosage of TFPI concentration in the CSF of CAA-ri patients

Having detected a decrease trend in the concentration of anti-Aβ antibodies in the CSF of CAA-ri patients during the remission phase of the disease (Figure 8b), the Inventors verified whether such trend were also confirmed for their endothelial damage biomarker, TFPI.

As it may be seen in Figure 14, TFPI values follow exactly the same trend previously found for autoantibodies (p = 0.013, apCAA-ri vs rpCAA-ri), as further evidence of the action mechanism previously described (Figure 8b). That is, autoantibodies act during the acute phases of the disease by removing the Aβ protein (Figures 10a and b), triggering adverse phenomena due to a rapid removal of vascular and parenchymal Aβ, in all likelihood caused by the high antibody concentrations detectable during apCAA-ri, analogously with what can be surmised during the appearance of VE (ARIA) in AD patients subjected to therapeutic immunisation.

Such evidences overall demonstrate that TFPI can be utilized as a novel valid endothelial damage biomarker consequent to amyloid deposition in neurodegenerative diseases and as an index for endothelial damage consequent to Aβ removal by anti-Aβ antibodies, both in spontaneous models of disease, such as CAA-ri, and for the follow-up of the therapeutic action during immunisation trials in AD or in disease-modifying therapies in general.

### 11. Analysis of diagnostic predictivity by ROC analysis and cut-off determination

As a last analysis, a statistical predictivity test was performed in order to evaluate the effectiveness of our markers for making a diagnosis of disease, by comparison of ROC curves for both of the biomarkers proposed (anti-Aβ antibodies and TFPI) on 25 healthy controls, compared to the 10 patients suffering from CAA-ri available to the Inventors.

We acted with two models:

### 1) ROC (Receiver Operating Characteristic) curve analysis for determining predictivity for an individual biomarker

### Biomarker 1: anti-Aβ antibodies

This test enabled us to demonstrate that the dosage of anti-Aβ antibodies in the CSF of apCAA-ri patients is highly predictive in discriminating the pathology (AUC= 0.97). From the analysis it was moreover possible to define a cut-off value, in terms of antibody concentration, below which the subject is not classifiable as suffering from CAA-ri. Said cut-off, for a 100% Sensitivity and a 90% Specificity set by the test, proved to be of <34.22 ng/ml.

### Biomarker 2: TFPI

This test enabled us to demonstrate that the dosage of TFPI in the CSF of apCAA-ri patients is highly predictive in discriminating the pathology (AUC= 0.99). From the analysis, moreover, it was possible to define a cut-off value, in terms of TFPI concentration, below which the subject is not classifiable as suffering from CAA-ri. Such a cut-off, for a 100% Sensitivity and an 89% Specificity set by the test, proved to be of <0.82 ng/mL.

### 2) Predictivity analysis by concomitantly utilizing both ROC curves

By an analysis which would concomitantly take into account values detectable for biomarker 1 and for biomarker 2, in the same patient, a sensitivity and a specificity close to 100% were found, confirming that a concomitant use of the two biomarkers allows a near-absolute ability to discriminate a CAA-ri patient from a healthy subject. In short, though reckoning that the case record at issue will necessarily have to be broadened and reconfirmed on a higher number of cases, by concomitantly evaluating the levels of TFPI and of anti-Aβ antibodies it is possible to provide a highly predictive diagnostic test, therefore advisable as a valid diagnostic tool, alternative to the cerebral biopsy still essential for a firm diagnosis of CAA-ri.

The same values of Specificity and Sensitivity were detected also after multivariate analysis in which we took into account other possible variables that might have affected the test, such as gender, age, tau and P-181 tau.

### REFERENCES

Y. Du, R. Dodel, H. Hampel, K. Buerger, S. Lin, B. Eastwood, K. Bales, F. Gao, H.J. Moeller, W. Oertel, M. Farlow, S. Paul, Reduced levels of amyloid betapeptide antibody in Alzheimer disease, Neurology 57(5) (2001) 801-805.
M.E. Weksler, N. Relkin, R. Turkenich, S. LaRusse, L. Zhou, P. Szabo, Patients with Alzheimer disease have lower levels of serum anti-amyloid peptide antibodies than healthy elderly individuals, Exp. Gerontol. 37(7) (2002) 943-948.
M.S. Song, I. Mook-Jung, H.J. Lee, J.Y. Min, M.H. Park, Serum anti-amyloid-beta antibodies and Alzheimer disease in elderly Korean patients, J. Int. Med. Res. 35(3) (2007) 301-306.
B.T. Hyman, C. Smith, I. Buldyrev, C. Whelan, H. Brown, M.X. Tang, R. Mayeux, Autoantibodies to amyloid-beta and Alzheimer disease, Ann. Neurol. 49(6) (2001) 808-810.
L. Baril, L. Nicolas, B. Croisile, P. Crozier, C. Hessler, A. Sassolas, J.B. McCormick, E. Trannoy, Immune response to Abeta-peptides in peripheral blood from patients with Alzheimer disease and control subjects, Neuroci. Lett. 355 (2004) 226-230.
A. Nath, E. Hall, M. Tuzova, M. Dobbs, M. Jons, C. Anderson, J. Woodward, Z. Guo, W. Fu, R. Kryscio, D. Wekstein, C. Smith, W.R. Markesbery, M.P. Mattson, Autoantobodies to amyloid betapeptide (Abeta) are increased in Alzheimer disease patients and Abeta antibodies can enhance Abeta neurotoxicity: implications for disease pathogenesis and vaccine development, Neuromol. Med. 3 (2003) 29-39.
Dodel R, Hampel H, Depboylu C, Lin S, Gao F, Schock S, Jäckel S, Wei X, Buerger K, Höft C, Hemmer B, Möller HJ, Farlow M, Oertel WH, Sommer N, Du Y. Human antibodies against amyloid beta peptide: a potential treatment for Alzheimer disease. Ann Neurol. 2002 Aug;52(2):253-6.
Di Francesco JC, Brioschi M, Brighina L, Ruffmann C, Saracchi E, Costantino G, Galimberti G, Conti E, Curtò NA, Marzorati L, Remida P, Tagliavini F, Savoiardo M, Ferrarese C, Anti-Aβ autoantibodies in the CSF of a patient with CAA-related inflammation: a case report. Neurology. 2011 Mar 1;76(9):842-4.
Conti E, Galimberti G, Piazza F, Raggi ME, Ferrarese C. Increased soluble APPalpha, Abeta 1-42, and anti-Abeta 1-42 antibodies in plasma from Down syndrome patients.
Alzheimer Dis Assoc Disord 2010;24:96 -100.

## Claims

1. An *in vitro* method for the quantification of antibodies anti-beta amyloid protein in a sample of cerebrospinal fluid comprising the following steps:
a) concentrating the quantity of said antibodies of said sample with magnetic micro beads coated with macromolecules capable of binding said antibodies;
b) analysing the concentrated sample obtained in step a) by immunoenzyme assay or by radioimmunoassay;
c) analysing a sample comprising antibodies anti-beta amyloid protein having a known titre with the same assay used in step b) and elaborating the related calibration curve, wherein said antibody having a known titre is a murine antibody belonging to the IgG2a or IgG2b class or a human or humanized antibody belonging to the IgG2b or IgG1 class wherein
said concentration step a) comprises a step of absorption of said cerebrospinal fluid sample on said micro beads and a step of elution of said antibodies from said micro beads;
said analysis step b) comprises the following steps:
I) providing a solid phase coated with beta amyloid protein and/or isoforms thereof;
II) incubating said solid phase with the concentrated material obtained from step a) thus allowing the binding of said autoantibodies to the beta amyloid protein on said solid phase , followed by rinsing to remove the unbound material;
III) incubating the solid phase obtained in step II) with a protein conjugated to an enzyme, or a radioactive label capable of binding human antibodies, followed by rinsing to remove said unbound conjugated protein;
IV) detecting the signal of said conjugated protein.

2. The method according to claim 1, wherein said antibody having a known titre is selected from: 4G8, 6 E10, G2-11, 3D6, m266, Solanezumab, Ponezumab, Bapineuzumab, Gantenerumab, BAN2401, BIIB037, Intravenously administered immunoglobulins (IVIG), Gammagard, Octagam, Newgam.

3. The method according to anyone of claims 1 to 2, wherein said antibody having a known titre is specific for an epitope comprised in the amino acid sequence in position 1-28 of the beta amyloid protein.

4. The method according to claim 3, wherein said antibody having a known titre recognises the epitope corresponding to the amino acid sequence in position 17-24 of the beta amyloid protein.

5. The method according to anyone of claims 1 to 4, wherein said conjugated protein is a protein conjugated to an enzyme, selected in the group comprising: Protein A-conjugated to Hrp, Protein G-conjugated to Hrp, conjugated biotin, conjugated streptavidin, antibodies conjugated to Hrp enzyme or to alkaline phosphatase (**ALP**) or to beta galactosidase or to a fluoresceinated molecule or to a luminescent molecule.

6. The method according to anyone of claims 1 to 6, wherein said protein conjugated to a radioactive label is selected in the group comprising: protein G conjugated to I-125, I-131 or I-99, A conjugated to I-125, I-131 or I-99, anti IgG antibody conjugated to I-125, I-131 or I-99.

7. An *in vitro* method for monitoring an active and/or passive immunisation treatment of the Alzheimer disease, wherein the steps of the method according to anyone of claims 1 to 6 are carried out on more CSF samples collected in different moments of said treatment and the quantification data obtained from each sample are compared with each other, thus constructing a progression in time of the concentration of antibodies against the beta amyloid protein in the CSF.

8. The method according to claim 7, further comprising a step of determining for each of said samples the concentration of one or more endothelial damage markers.

9. The method according to claim 8, wherein said endothelial damage marker is the tissue factor pathway inhibitor (TFPI).

10. An *in vitro* method for the diagnosis or the therapeutic monitoring of cerebral amyloid angiopathy (CAA), even associated to inflammations and vasogenic oedema (CAA-ri), from a CSF sample of a patient comprising the steps of the method according to anyone of claims 1 to 8 and a further step of comparing the determined concentration of antibodies anti-beta amyloid protein with a standard reference value.

11. The method according to claim 10, wherein said standard reference value is determined by a ROC curve obtained by elaborating the concentrations of a set of samples from healthy individuals and individuals suffering from CAA or CAA-ri.

12. The method according to claim 10 or 11, wherein said reference value is comprised between 30 and 40 ng/mL.

13. The method according to anyone of claims 10 to 12, comprising a further step of determining the concentration of TFPI in a CSF sample of said patient and of comparing the determined value with a standard reference value.

14. The method according to claim 13, wherein said standard reference value of TFPI is determined by a ROC curve obtained by elaborating the concentrations of a set of samples from healthy individuals and individuals suffering from CAA or CAA-ri.

15. The method according to claim 13 or 14, wherein said standard reference value of TFPI is comprised between 0.70 and 0.90 ng/mL.

16. An *in vitro* method for the diagnosis or the therapeutic monitoring of radiological abnormalities (ARIA) from a CSF sample of a patient comprising the steps of the method according to anyone of claims 1 to 6 and a further step of comparing the determined concentration of antibodies anti-beta amyloid protein with a standard reference value.

17. The method according to claim 16, comprising a further step of determining the concentration of TFPI in a CSF sample of said patient and of comparing the determined value with a standard reference value.

18. The method according to claim 17, wherein said standard reference value of TFPI is determined by a ROC curve obtained by elaborating the concentrations of a set of samples from healthy individuals and individuals suffering from CAA or CAA-ri.

19. The method according to claim 18 or 18, wherein said standard reference value of TFPI is comprised between 0.70 and 0.90 ng/mL.

20. A kit for the detection of antibodies of beta amyloid protein comprising magnetic micro beads coated with macromolecules capable of binding said antibodies and one or more aliquots of antibodies having a known titre against beta amyloid protein, wherein said antibody having a known titre is a murine antibody belonging to the IgG2a or IgG2b class or a human antibody belonging to the IgG2b or IgG1 class.

## Patentansprüche

1. *In-vitro-Verfahren* zur Quantifizierung von anti-Beta-Amyloid-Protein-Antikörpern in einer Liquor-Probe, das die folgenden Schritte umfasst:
a) Konzentrieren der Menge der Antikörper der Probe mit magnetischen Mikroperlen, die mit Makromolekülen beschichtet sind, die in der Lage sind, die Antikörper zu binden;
b) Analysieren der in Schritt a) erhaltenen konzentrierten Probe durch ein Immunoenzymassay oder ein Radioimmunassay;
c) Analysieren einer Probe, die anti-Beta-Amyloid-Protein-Antikörper mit einem bekannten Titer umfasst, mit demselben Assay, das in Schritt b) verwendet wird, und Ausarbeiten der dazugehörigen Kalibrierkurve, wobei der Antikörper mit einem bekannten Titer ein muriner Antikörper ist, der zu der IgG2a- oder IgG2b-Klasse gehört, oder ein menschlicher oder humanisierter Antikörper ist, der zu der IgG2b- oder IgG1-Klasse gehört, wobei
der Konzentrierungsschritt a) einen Schritt zur Absorption der Liquor-Probe auf den Mikroperlen und einen Schritt einer Elution der Antikörper von den Mikroperlen umfasst;
der Analyseschritt b) die folgenden Schritte umfasst:
I) Bereitstellen einer Festphase, die mit Beta-Amyloid-Protein und/oder Isoformen davon beschichtet ist;
II) Inkubieren der Festphase mit dem aus Schritt a) erhaltenen konzentrierten Material, wodurch das Binden der Autoantikörper an das Beta-Amyloid-Protein auf der Festphase ermöglicht wird, gefolgt durch Spülen zur Entfernung des ungebundenen Materials;
III) Inkubieren der in Schritt II) erhaltenen Festphase mit einem an ein Enzym konjugiertes Protein, oder einem radioaktiven Marker, der in der Lage ist menschliche Antikörper zu binden, gefolgt durch Spülen zur Entfernung des ungebundenen konjugierten Proteins;
IV) Erfassen des Signals des konjugierten Proteins.

2. Verfahren gemäß Anspruch 1, wobei der Antikörper mit einem bekannten Titer aus der folgenden Gruppe ausgewählt wird: 4G8, 6 E10, G2-11, 3D6, m266, Solanezumab, Ponezumab, Bapineuzumab, Gantenerumab, BAN2401, BIIB037, intravenös verabreichte Immunoglobuline (IVIG), Gammagard, Octagam, Newgam.

3. Verfahren gemäß einem der Ansprüche 1 bis 2, wobei der Antikörper mit einem bekannten Titer für ein Epitop spezifisch ist, das in der Aminosäuresequenz in Position 1-28 des Beta-Amyloid-Proteins enthalten ist.

4. Verfahren gemäß Anspruch 3, wobei der Antikörper mit einem bekannten Titer das Epitop entsprechend der Aminosäuresequenz in Position 17-24 des Beta-Amyloid-Proteins erkennt.

5. Verfahren gemäß einem der Ansprüche 1 bis 4, wobei das konjugierte Protein ein an ein Enzym konjugiertes Protein ist, das aus der folgenden Gruppe ausgewählt wird: an Hrp konjugiertes Protein A, an Hrp konjugiertes Protein G, konjugiertes Biotin, konjugiertes Streptavidin, an Hrp-Enzym oder an alkalische Phosphatase (ALP) oder an Beta-Galaktosidase oder an ein fluoreszierendes Molekül oder an ein lumineszierendes Molekül konjugierter Antikörper.

6. Verfahren gemäß einem der Ansprüche 1 bis 6, wobei das an einen radioaktiven Marker konjugierte Protein aus der folgenden Gruppe ausgewählt wird: an I-125, I-131 oder I-99 konjugiertes Protein G, an I-125, I-131 oder I-99 konjugiertes Protein A, an I-125, I-131 oder I-99 konjugierter anti-IgG-Antikörper.

7. *In-vitro-Verfahren* zum Überwachen einer aktiven und/oder passiven Immunisierungsbehandlung der Alzheimer Krankheit, wobei die Schritte des Verfahrens gemäß einem der Ansprüche 1 bis 6 mit mehreren CSF-Proben ausgeführt werden, die in unterschiedlichen Augenblicken der Behandlung gewonnen werden, und die Quantifizierungsdaten, die aus jeder Probe erhalten werden, miteinander verglichen werden, wodurch ein Zeitverlauf der Konzentration von Antikörpern gegen das Beta-Amyloid-Protein in dem CSF konstruiert wird.

8. Verfahren gemäß Anspruch 7, das weiterhin einen Schritt zum Bestimmen, für jede der Proben, der Konzentration von einem oder mehreren Markern für Endothelschäden umfasst.

9. Verfahren gemäß Anspruch 8, wobei der Marker für Endothelschäden der Tissue-Factor-Pathway-Inhibitor (TFPI) ist.

10. *In-vitro-Verfahren* zur Diagnose oder therapeutischen Überwachung von zerebraler Amyloidangiopathie (CAA), gleichermaßen verknüpft mit Entzündungen und vasogenem Ödem (CAA-ri), aus einer CSF-Probe eines Patienten, das umfasst: die Schritte des Verfahrens gemäß einem der Ansprüche 1 bis 8 und einen weiteren Schritt eines Vergleichens der bestimmten Konzentration von anti-Beta-Amyloid-Protein-Antikörpern mit einem Standardreferenzwert.

11. Verfahren gemäß Anspruch 10, wobei der Standardreferenzwert durch eine ROC-Kurve bestimmt wird, die durch Erarbeiten der Konzentrationen eines Satzes von Proben von gesunden Individuen und Individuen, die an CAA oder CAA-ri leiden, erhalten wird.

12. Verfahren gemäß Anspruch 10 oder 11, wobei der Referenzwert zwischen 30 und 40 ng/ml liegt.

13. Verfahren gemäß einem der Ansprüche 10 bis 12, das einen weiteren Schritt zum Bestimmen der Konzentration von TFPI in einer CSF-Probe des Patienten und zum Vergleichen des bestimmten Wertes mit einem Standardreferenzwert umfasst.

14. Verfahren gemäß Anspruch 13, wobei der Standardreferenzwert von TFPI durch eine ROC-Kurve bestimmt wird, die durch ein Erarbeiten der Konzentrationen eines Satzes von Proben von gesunden Individuen und Individuen, die an CAA oder CAA-ri leiden, erhalten wird.

15. Verfahren gemäß Anspruch 13 oder 14, wobei der Standardreferenzwert von TFPI zwischen 0,70 und 0,90 ng/ml liegt.

16. *In-vitro-Verfahren* zur Diagnose oder therapeutischen Überwachung von radiologischen Anomalien (ARIA) aus einer CSF-Probe eines Patienten, das umfasst: die Schritte des Verfahrens gemäß einem der Ansprüche 1 bis 6 und einen weiteren Schritt zum Vergleichen der bestimmten Konzentration von anti-Beta-Amyloid-Protein-Antikörpern mit einem Standardreferenzwert.

17. Verfahren gemäß Anspruch 16, das einen weiteren Schritt zum Bestimmen der Konzentration von TFPI in einer CSF-Probe des Patienten und zum Vergleichen des bestimmten Wertes mit einem Standardreferenzwert umfasst.

18. Verfahren gemäß Anspruch 17, wobei der Standardreferenzwert von TFPI durch eine ROC-Kurve bestimmt wird, die durch ein Erarbeiten der Konzentrationen eines Satzes von Proben von gesunden Individuen und Individuen, die unter CAA oder CAA-ri leiden, erhalten wird.

19. Verfahren gemäß Anspruch 18 oder 18, wobei der Standardreferenzwert von TFPI zwischen 0,70 und 0,90 ng/ml liegt.

20. Kit zur Detektion von Beta-Amyloid-Protein-Antikörpern, das umfasst: magnetische Mikroperlen, die mit Makromolekülen beschichtet sind, die in der Lage sind, die Antikörper zu binden, und eine oder mehrere Aliquote von Antikörpern mit einem bekannten Titer gegen Beta-Amyloid-Protein, wobei der Antikörper mit einem bekannten Titer ein muriner Antikörper ist, der zu der IgG2a- oder IgG2b-Klasse gehört, oder ein menschlicher Antikörper ist, der zu der IgG2b- oder IgG1-Klasse gehört.

## Revendications

1. Procédé *in vitro* de quantification des anticorps anti-protéine bêta-amyloïde dans un échantillon de liquide céphalo-rachidien comprenant les étapes suivantes :
a) la concentration de la quantité desdits anticorps dudit échantillon avec des microbilles magnétiques revêtues de macromolécules capables de lier lesdits anticorps ;
b) l'analyse de l'échantillon concentré obtenu dans l'étape a) par un test immunoenzymatique ou par un test radioimmunologique ;
c) l'analyse d'un échantillon comprenant des anticorps anti-protéine bêta-amyloïde ayant un titre connu avec le même test que celui utilisé dans l'étape b) et l'élaboration de la courbe d'étalonnage associée, dans lequel ledit anticorps ayant un titre connu est un anticorps murin appartenant à la classe des IgG2a ou des IgG2b ou un anticorps humain ou humanisé appartenant à la classe des IgG2b ou des IgG1 dans lequel
chaque étape de concentration a) comprend une étape d'absorption dudit échantillon de liquide céphalo-rachidien sur lesdites microbilles et une étape d'élution desdits anticorps à partir desdites microbilles ;
ladite étape d'analyse b) comprend les étapes suivantes :
I) la fourniture d'une phase solide revêtue de protéine bêta-amyloïde et/ou de ses isoformes ;
II) l'incubation de ladite phase solide avec le matériau concentré obtenu à partir de l'étape a) permettant ainsi la liaison desdits auto-anticorps à la protéine bêta-amyloïde sur ladite phase solide, suivie d'un rinçage pour éliminer le matériau non lié ;
III) l'incubation de la phase solide obtenue dans l'étape II) avec une protéine conjuguée à une enzyme, ou un marqueur radioactif capable de lier des anticorps humains, suivie d'un rinçage pour éliminer ladite protéine conjuguée non liée ;
IV) la détection du signal de ladite protéine conjuguée.

2. Procédé selon la revendication 1, dans lequel ledit anticorps ayant un titre connu est choisi parmi : 4G8, 6 E10, G2-11, 3D6, m266, solanezumab, ponezumab, bapineuzumab, ganténérumab, BAN2401, BIIB037, immunoglobulines administrées par voie intraveineuse (IgIV), Gammagard, Octagam, Newgam.

3. Procédé selon l'une quelconque des revendications 1 ou 2, dans lequel ledit anticorps ayant un titre connu est spécifique d'un épitope compris dans la séquence d'acides aminés en position 1-28 de la protéine bêta-amyloïde.

4. Procédé selon la revendication 3, dans lequel ledit anticorps ayant un titre connu reconnaît l'épitope correspondant à la séquence d'acides aminés en position 17-24 de la protéine béta-amyloïde.

5. Procédé selon l'une quelconque des revendications 1 à 4, dans lequel ladite protéine conjuguée est une protéine conjuguée à une enzyme, choisie dans le groupe comprenant la protéine A conjuguée à la HRP, la protéine G conjuguée à la HRP, la biotine conjuguée, la streptavidine conjuguée, des anticorps conjugués à l'enzyme HRP ou à la phosphatase alcaline (PAL) ou à la bêta-galactosidase ou à une molécule à base de fluorescéine ou à une molécule luminescente.

6. Procédé selon l'une quelconque des revendications 1 à 6, dans lequel ladite protéine conjuguée à un marqueur radioactif est choisie dans le groupe comprenant: la protéine G conjuguée à I-125, I-131 ou I-99, la protéine A conjuguée à I-125, I-131 ou I-99, un anticorps anti-IgG conjugué à I-125, I-131 ou I-99.

7. Procédé *in vitro* de surveillance d'un traitement d'immunisation active et/ou passive de la maladie d'Alzheimer, dans lequel les étapes du procédé selon l'une quelconque des revendications 1 à 6 sont réalisées sur plus d'échantillons de LCR prélevés à des moments différents dudit traitement et les données de quantification obtenues de chaque échantillon sont comparées les unes aux autres, construisant ainsi une progression dans le temps de la concentration des anticorps dirigés contre la protéine bêta-amyloïde dans le LCR.

8. Procédé selon la revendication 7, comprenant en outre une étape de détermination pour chacun desdits échantillons de la concentration d'un ou de plusieurs marqueurs de la lésion endothéliale.

9. Procédé selon la revendication 8, dans lequel ledit marqueur de la lésion endothéliale est l'inhibiteur de la voie du facteur tissulaire (TFPI).

10. Procédé *in vitro* de diagnostic ou de surveillance thérapeutique de l'angiopathie amyloïde cérébrale (AAC), même associée à des inflammations et un oedème vasogénique (AAC-I), à partir d'un échantillon de LCR d'un patient comprenant les étapes du procédé selon l'une quelconque des revendications 1 à 8 et une étape supplémentaire de comparaison de la concentration déterminée des anticorps anti-protéine bêta-amyloïde à une valeur de référence classique.

11. Procédé selon la revendication 10, dans lequel ladite valeur de référence classique est déterminée par une courbe ROC obtenue en élaborant les concentrations d'un ensemble d'échantillons provenant d'individus en bonne santé et d'individus souffrant d'AAC ou d'AAC-I.

12. Procédé selon la revendication 10 ou 11, dans lequel ladite valeur de référence est comprise entre 30 et 40 ng/ml.

13. Procédé selon l'une quelconque des revendications 10 à 12, comprenant une étape supplémentaire de détermination de la concentration du TFPI dans un échantillon de LCR dudit patient et de comparaison de la valeur déterminée à une valeur de référence classique.

14. Procédé selon la revendication 13, dans lequel ladite valeur de référence classique du TFPI est déterminée par une courbe ROC obtenue en élaborant les concentrations d'un ensemble d'échantillons provenant d'individus en bonne santé et d'individus souffrant d'AAC ou d'AAC-I.

15. Procédé selon la revendication 13 ou 14, dans lequel ladite valeur de référence classique du TFPI est comprise entre 0,70 et 0,90 ng/ml.

16. Procédé *in vitro* de diagnostic ou de surveillance thérapeutique d'anomalies radiologiques (ARIA) à partir d'un échantillon de LCR d'un patient comprenant les étapes du procédé selon l'une quelconque des revendications 1 à 6 et une étape supplémentaire de comparaison de la concentration déterminée des anticorps anti-protéine bêta-amyloïde à une valeur de référence classique.

17. Procédé selon la revendication 16, comprenant une étape supplémentaire de détermination de la concentration du TFPI dans un échantillon de LCR dudit patient et de comparaison de la valeur déterminée à une valeur de référence classique.

18. Procédé selon la revendication 17, dans lequel ladite valeur de référence classique du TFPI est déterminée par une courbe ROC obtenue en élaborant les concentrations d'un ensemble d'échantillons provenant d'individus en bonne santé et d'individus souffrant d'AAC ou d'AAC-I.

19. Procédé selon la revendication 18 ou 18, dans lequel ladite valeur de référence classique du TFPI est comprise entre 0,70 et 0,90 ng/ml.

20. Kit de détection d'anticorps de la protéine bêta-amyloïde comprenant des microbilles magnétiques revêtues de macromolécules capables de lier lesdits anticorps et une ou plusieurs aliquotes d'anticorps ayant un titre connu contre la protéine bêta-amyloïde, dans lequel ledit anticorps ayant un titre connu est un anticorps murin appartenant à la classe des IgG2a ou des IgG2b ou un anticorps humain appartenant à la classe des IgG2b ou des IgG1.
